# EUROPÄISCHE PATENTSCHRIFT

(11) **EP 4 103 275 B1**
(45) Veröffentlichungstag und Bekanntmachung des Hinweises auf die Patenterteilung: **08.07.2026**
(21) Anmeldenummer: 21708917.6
(22) Anmeldetag: 12.02.2021
(51) Int. Cl.: A61N 5/06

(54) **KÖRPERBESTRAHLUNGSVORRICHTUNG**
BODY IRRADIATING DEVICE
DISPOSITIF D'IRRADIATION DU CORPS

(30) Priorität: 12.02.2020 DE 202020100756 U
(43) Veröffentlichungstag der Anmeldung: 21.12.2022
(73) Patentinhaber: JK-Holding GmbH, 53578 Windhagen (DE)
(72) Erfinder: GERSTENMEIER, Jürgen, 53578 Windhagen (DE)
(74) Vertreter: IPrime Bonnekamp Sparing Patentanwaltsgesellschaft mbH
(86) Internationale Anmeldenummer: PCT/DE2021/100143
(87) Internationale Veröffentlichungsnummer: WO 2021/160223

(56) Entgegenhaltungen:
- EP-A1- 3 597 268
- WO-A1-2007/114614
- US-A1- 2005 065 579
- US-A1- 2016 276 549
- US-A1- 2019 074 411

## Beschreibung

Die Erfindung betrifft eine Körperbestrahlungsvorrichtung nach dem Oberbegriff des unabhängigen Anspruchs.

Aus der Praxis sind Körperbestrahlungsvorrichtungen für den menschlichen Körper bekannt, die insbesondere als Bestrahlungseinrichtung in Gestalt eines Solariums mit Liegefläche oder eines Stehbräuners oder einer Rotlichtbehandlungsliege ausgebildet sind, bei denen der menschliche Körper oder Teile des menschlichen Körpers mit einem Strahlungsspektrum in bestimmten Wellenlängenbereichen beaufschlagt wird, um das Wohlbefinden, die Gesundheit oder die Regeneration des menschlichen Körpers zu beeinflussen. Die bekannten Körperbestrahlungsvorrichtungen setzen hierbei teils Niederdruckstrahlungsröhren, teils Hochdruckstrahlungsröhren ein, die ein breites Strahlungsspektrum emittieren, und bei denen es gegebenenfalls erforderlich ist, einen Filter anzuordnen, um schädliche Strahlung vom Erreichen der Oberfläche des menschlichen Körpers zu verhindern. Nachteilig bei den Körperbestrahlungsvorrichtungen mit Niederdruck- oder Hochdruckröhren ist insbesondere, dass die Strahlungsintensität über das breite Strahlungsspektrum stark schwankt, sodass die richtige Dosierung der Strahlung sowie das Beaufschlagen mit gewünschten, besonders günstigen Strahlungspeaks entweder nicht erreicht wird oder eine hohe Verluststrahlung nach sich zieht. Insbesondere Kombinationsbehandlungen, bei denen bestimmte Bestrahlungspeaks in Kombination den Körper beaufschlagen sollen, stellen sich dabei allenfalls zufällig ein und können in ihrer relative Intensität kaum beeinflusst werden.

In der Praxis sind Körperbestrahlungsvorrichtungen vorgeschlagen worden, bei denen jeweils ein LED-Chip unter einer Linse angeordnet ist und ein relativ schmales Strahlungsspektrum mit einem vergleichsweise definierten Strahlungspeak emittiert, wobei die Schwierigkeit hier darin besteht, den menschlichen Körper mit gleichmäßiger Strahlung unterschiedlicher Wellenlängen zu beaufschlagen, sodass über die gesamte Oberfläche des menschlichen Körpers eine homogene Beaufschlagung erfolgt.

EP 2 800 605 B1 beschreibt eine Körperbestrahlungsvorrichtung zur Unterstützung der Bildung von Vitamin D, bei der verschiedene Lichtquellen, darunter auch eine Vielzahl von LEDs, angegeben sind, die ein Strahlungsspektrum aussenden können, wobei die Lichtquelle in einem als Hohlspiegel ausgebildeten Reflektor angeordnet ist und die von der Lichtquelle ausgesandte Strahlung durch den Reflektor kollimiert wird, bevor sie durch eine Filterlage, die eine vorgegebene Bandbreite der Strahlung passieren lässt, hindurchtritt. Nachteilig ist hierbei, dass keine gleichmäßige Bestrahlung unter definierte Abstimmung mehrerer Peaks innerhalb des Strahlungsspektrums möglich ist. Nachteilig ist ferner, dass ein großer Bauraum benötigt wird. Ferner bewirkt die Auslöschung bestimmter Wellenlängen durch die Filterlage, dass ein Teil der Energie ungenutzt bleibt.

US 2005 006 55 79 A1 zeigt eine Körperbestrahlungsvorrichtung, die es ermöglicht, den Körper einer Person oder einen Teil hiervon mit Strahlung, insbesondere Infrarot-Strahlung, zu beaufschlagen, wobei die Vorrichtung als handbetätigtes Gerät ausgebildet ist und eine Schaltkreisplatine aufweist. Mit der Schaltkreisplatine ist über eine Leiterbahn eine Beleuchtungsanordnung verbunden, die eine oder mehrere ersetzbare lichtemittierende Elemente, insbesondere LEDs umfasst. Diese lichtemittierende Anordnung stellt eine Bestrahlungsquelle dar, die inhärent eine Basis aufweist und für jedes ersetzbare lichtemittierende Element eine LED und damit auch einen Chip umfasst, wobei der LED-Chip ein Strahlungsspektrum mit einem Strahlungspeak aussenden kann. Innerhalb der lichtemittierenden Anordnung sind weitere lichtemittierende Elemente angeordnet, die ebenfalls jeweils einen Strahlungspeak aufweisen und deren Strahlungsspektrum sich von demjenigen des ersten LED-Chips unterscheiden. Die gesamte lichtemittierende Anordnung wird von einer Linse 102 überdeckt, die eine transparente Abdeckung, nicht aber eine optische Linse darstellt.

US 2020 000 11 05 A1 zeigt eine als Bräunungsvorrichtung ausgebildete Körperbestrahlungsvorrichtung, die zur Bestrahlung eines Körpers einer Person bzw. von Teilen hiervon mit Strahlung mit im einzelnen angegebenen Wellenlängen ausgebildet ist, bei der Bestrahlungsquellen vorgesehen sind, die eine Mehrzahl von LEDs aufweisen, wobei die LEDs verschiedene Strahlungsspektren aussenden können, wobei die Ausgestaltung der Optik und die bauliche Anordnung der LEDs konventionell ausgebildet ist.

US 2012 004 39 07 A1 beschreibt eine Bestrahlungsvorrichtung zur Bestrahlung von Pflanzen, die eine Bestrahlungsquelle mit einer Basis, zumindest einem ersten LED-Chip und einem zweiten LED-Chip umfasst, wobei der erste LED-Chip ein von dem zweiten LED-Chip verschiedenes Strahlungsspektrum größer als 400 nm, konkret beispielsweise rot und blau, im sichtbaren Spektrum aussendet, sodass deren recht breitbandigen Strahlungspeak jeweils unterschiedlich sind. Der erste LED-Chip und zweite LED-Chip sind unter einer gemeinsamen Linse in einem LED-Gehäuse eingerichtet und sind auch separat ansteuerbar. Die Bestrahlungsvorrichtung ist nicht zur Bestrahlung eines menschlichen Körpers oder eines Teils hiervon ausgebildet oder geeignet, da in einem vergleichsweise großen Abstand zu den Pflanzen eine lang andauernde Bestrahlung mit entsprechend geringer Intensität erfolgt; wird die Bestrahlungsvorrichtung näher an das Bestrahlungsziel gerückt, entstehen abgeschattete Bereiche.

US 2015 009 72 00 A1 beschreibt eine Bestrahlungsvorrichtung, um insbesondere sichtbares Licht mit einer Kombination von LEDs und einem fluoreszierenden Material zu erzeugen, dessen Strahlungssprektrum entsprechend breit und vergleichsweise gleichmäßig ausfällt. Die Bestrahlungsvorrichtung weist eine Basis auf, auf der ein erster LED-Chip, ein zweiter LED-Chip, ein dritter LED-Chip, ein vierter LED-Chip angeordnet sind, deren Strahlungsspektrum verschieden ausgebildet sein kann, wobei die LED-Chips unter einer gemeinsamen Linse in einem LED-Gehäuse angeordnet sind, und die LED-Chips hierbei separat ansteuerbar sind. Der von dem ersten LED-Chip erzeugte erste Strahlungspeak liegt zwischen 430 nm und 455 nm bzw. 479 nm, und der von dem zweiten LED-Chip erzeugte zweite Strahlungspeak liegt zwischen 456 nm und 469 nm bzw. 600 nm bis 650 nm, wobei ein von einem dritten LED-Chip erzeugter erste Strahlungspeak zwischen in der ersten Alternative zwischen 600 nm und 650 nm liegt. Hierbei strahlt das fluoreszierende bzw. anregbare Material Strahlung mit einem Strahlungspeak zwischen 500 nm und 555 nm aus, wobei die Strahlung der LEDs und die Strahlung des fluoreszierenden Materials sich überlagert. Ein Einsatz nur der LEDs ohne fluoreszierendes Material ist nicht vorgesehen. Verwendung findet die Bestrahlungsvorrichtung beispielsweise als Tischlampe oder dergleichen, nicht aber zur Bestrahlung des menschlichen Körpers. Weiter wird ein Verfahren zur Herstellung einer Bestrahlungsvorrichtung beschrieben.

US 2019 007 44 11 A1 beschreibt eine Bestrahlungsvorrichtung, bei der mehrere LED-Chips in einer Kavität angeordnet sind, wobei der nicht von den LED-Chips ausgefüllte Bereicht mit einer fluoreszierenden Substanz ausgefüllt ist, wobei die fluoreszierende Substanz die Strahlung der LED-Chips teilweise in Strahlung mit einer von der Strahlung der LED-Chips verschiedenen sekundären Strahlung umwandelt, und ein weißes Licht erzeugt wird.

WO 2007 114 614 A1 beschreibt eine Bestrahlungsvorrichtung, die Strahlung im sichtbaren Spektrum emitiert, wobei zur Erzeugung einer dem Sonnenlicht vergleichbaren Strahlung eine Bestrahlungsquelle mit einer Basis und zumindest einem auf der Basis angeordneten ersten LED-Chip, der ein erstes Strahlungsspektrum mit einem ersten Strahlungspeak aussenden kann, und zumindest einem zweiten LED-Chip, der ein zweites Strahlungsspektrum mit einem zweiten Strahlungspeak aussenden kann, vorgesehen ist. Der erste LED-Chip ist von einem eine erste Linse ausbildenden Duroplast-Kunstharz mit darin enthalten fluoreszierenden Material überzogen, der zweite LED-Chip ist von einem eine zweite Linse ausbildenden Duroplast-Kunstharz mit darin enthaltenen fluoreszierendem Material überzogen. Die LED-Chips mit ihren respektiven fluoreszierenden Abdeckungen sind in einem Ausführungsbeispiel in einer Kavität nebeneinander untergebracht. In einem anderen Ausführungsbeispiel sind die LED-Chips mit ihren respektiven fluoreszierenden Abdeckungen nebeneinander angeordnet und von einem linsenartigen geformten Abschnitt umgeben. Die LED-Chips sind separat ansteuerbar.

US 2016 027 65 49 A1 beschreibt eine als LED ausgebildete Strahlungsquelle, umfassend einen ersten LED-Chip und einen zweiten LED-Chip, die in benachbarten LED-Gehäusen oder zumindest in einem LED-Gehäuse mit einer zwischen den LED-Chips angeordneten Trennwand angeordnet sind. Die jeweils die LED-Chips enthaltenden LED-Gehäuse sind mit einem Kunstharz verfüllt, in dem fluoreszierende Materialien eingebettet sind, sodass die von den LED-Chips emitierte Primärstrahlung mit einer Wellenlänge von 430 nm - 480 nm bzw. nahe 450 nm durch die fluoreszierende Materialien in Strahlung größerer Wellenlängen umgewandelt wird. Die LED-Chips lassen sich hierbei unabhängig voneinander regeln. Im Ergebnis erzeugt die Strahlungsquelle eine aus einer ersten weißen Strahlung und einer zweiten weißen Strahlung gemischte weiße Strahlung. Der Strahlungsquelle ist eine flache Lichtleitplatte mit rechteckigem Grundriss aus transparentem Material nachgeordnet, die die in eine Schmalseite eingeleitete Strahlung an den hierzu senkrechten Flächen austreten lässt.

US 2020 000 11 05 A1 (nächstkommender Stand der Technik) zeigt eine Körperbestrahlungsvorrichtung zur Bestrahlung eines Körpers einer Person oder eines Teils eines Körpers einer Person, die nach Art eines Solariums ausgestaltet ist. Die Körperbestrahlungsvorrichtung umfasst ein Gehäuse, das einen tunnelartigen Bestrahlungsraum für die zu bestrahlende Person zumindest teilweise umgibt und das zumindest einen Aufnahmeraum für Bestrahlungsquellen aufweist, wobei die Strahlung der Bestrahlungsquellen aus dem Aufnahmeraum in den Bestrahlungsraum gerichtet ist. Die Bestrahlungsquelle umfasst eine Mehrzahl von LEDs, die jeweils voneinander verschiedene Strahlungsspektren mit einem voneinander verschiedenen Strahlungspeak aussenden können und die separat ansteuerbar sind.

Es ist die Aufgabe der Erfindung, eine Körperbestrahlungsvorrichtung anzugeben, mit der das Strahlungsspektrum von mehr als einem LED-Chip kombiniert werden und gleichmäßig den menschlichen Körper beaufschlagen kann.

Diese Aufgabe wird erfindungsgemäß durch eine Körperbestrahlungsvorrichtung mit den Merkmalen des unabhängigen Patentanspruchs gelöst.

Gemäß einem Aspekt der Erfindung ist eine Körperbestrahlungsvorrichtung geschaffen, umfassend eine Bestrahlungsquelle mit einer Basis und mit zumindest einem ersten LED-Chip, der ein erstes Strahlungsspektrum mit einem ersten Strahlungspeak aussenden kann, und mit zumindest einem zweiten LED-Chip, der ein zweites Strahlungsspektrum mit einem von dem ersten Strahlungspeak verschiedenen zweiten Strahlungspeak aussenden kann, die sich weiter dadurch auszeichnet, dass der erste LED-Chip und der zweite LED-Chip unter einer gemeinsamen Linse in einem LED-Gehäuse angeordnet sind und separat ansteuerbar sind. Durch das Anordnen des ersten LED-Chips und des zweiten LED-Chips unter der gemeinsamen Linse kann Strahlung mit dem ersten Strahlungspeak und Strahlung mit dem zweiten Strahlungspeak gleichmäßig durch die gemeinsame Linse hindurch ausgesendet werden - vorzugsweise mit nachgeschaltetem Kollimations-Reflektor zum Kollimieren des ersten Strahlungsspektrums und des zweiten Strahlungsspektrum - , sodass der nachgeordnete Bereich, in dem ein menschlicher Körper liegen kann, gleichmäßig mit dem ersten Strahlungsspektrum und dem zweiten Strahlungsspektrum ausgeleuchtet wird. Hierdurch wird eine besonders homogene Verteilung der Strahlung erreicht und insbesondere vermieden, dass Intensitätsspitzen den menschlichen Körper erreichen. Weiterhin kann durch das separate Ansteuern des ersten LED-Chips und des zweiten LED-Chips die Strahlungsintensität des ersten Strahlungsspektrums und des zweiten Strahlungsspektrums individuell angepasst und geregelt werden, sodass nicht nur die simultane Bestrahlung mit dem ersten Strahlungsspektrum und dem zweiten Strahlungsspektrum ermöglicht ist, sondern darüber hinaus eine intensitätsmäßige Abstimmung der Peaks aufeinander in Hinblick auf das gewünschte Ergebnis. Die separate Ansteuerbarkeit der LED-Chips umfasst besonders bevorzugt die Möglichkeit, die Intensität der Strahlung der verschiedenen LED-Chips einzeln und unabhängig voneinander einzeln einzustellen, wodurch sich günstige Peak-Verhältnisse einstellen lassen, die zugleich den Energieverbrauch günstig reduzieren. Überdies kann vorgesehen sein, dass die LED-Chips auch separat kontaktierbar sind. Die LED-Chips sind hierbei auf der Basis angeordnet.

Die erfindungsgemäße Körperbestrahlungsvorrichtung umfasst hierbei ein Gehäuse, das einen tunnelartigen Bestrahlungsraum für eine zu bestrahlende Person zumindest teilweise umgibt und das zumindest einen Aufnahmeraum für die Lichtquellen aufweist. Eine Trennwand des Gehäuses zwischen dem Aufnahmeraum und dem Bestrahlungsraum ist aus einem für die Strahlung der Bestrahlungsquellen transparenten Material ausgebildet. Die Strahlung der Bestrahlungsquellen ist hierbei aus dem Aufnahmeraum in den Bestrahlungsraum gerichtet.

Das LED-Gehäuse ist hierbei dasjenige Gehäuse, in dem die LED-Chips untergebracht sind. Es handelt sich damit eigentlich um ein LED-Chip-Gehäuse. Nicht mit LED-Gehäuse ist ein Gehäuse gemeint, in dem eine oder mehrere LEDs untergebracht sind, die selbst wiederum in einem LED-Gehäuse aufgenommene LED-Chips enthalten. Das LED-Gehäuse ist mit Kontakten ausgestattet, um die einzelnen LED-Chips zu kontaktieren. Die Linse, die das LED-Gehäuse bedeckt, schließt den oder die in dem LED-Gehäuse aufgenommenen LED-Chips nach außen ab, sodass die resultierende LED entsprechend einsetzbar ist.

Die Körperbestrahlungsvorrichtung ist ausgebildet zur Bestrahlung eines menschlichen Körpers einer Person oder eines Teils eines menschlichen Körpers einer Person, also des Leibes eines Lebewesens bzw. eines Individuums, einschließlich aller Gliedmaßen und Oberflächenbereiche, insbesondere mit kosmetisch-hygienisch nützlicher Strahlung. Hierdurch unterscheidet sie sich von Bestrahlungsvorrichtungen, die Strahlung auf Gegenstände richten und bei denen keine gesundheitlichen Einschränkungen hinsichtlich beispielsweise Dosis und Strahlungsspektrum zu beachten sind. Somit können als Körper insbesondere einzelne oder alle Gliedmaßen einer menschlichen Person oder gegebenenfalls eines anderen Säugers angesehen werden.

Vorzugsweise umfasst die Bestrahlungsquelle zumindest einen dritten LED-Chip, der ein drittes Strahlungsspektrum mit einem von dem ersten Strahlungspeak und dem zweiten Strahlungspeak verschiedenen dritten Strahlungspeak aussenden kann, wobei der dritte LED-Chip mit dem zumindest einen ersten LED-Chip und dem zumindest einen zweiten LED-Chip unter der gemeinsamen Linse in einem LED-Gehäuse angeordnet ist und separat von diesen ansteuerbar ist. Hierdurch ist es vorteilhaft möglich, auch ein drittes Strahlungsspektrum mit einem dritten Strahlungspeak homogen und gleichmäßig sowie intensitätsunabhängig von den anderen beiden Strahlungspeaks auf den menschlichen Körper zu richten, und damit drei Strahlungsspektren mit drei verschiedenen Strahlungspeaks gleichmäßig und intensitätsmäßig abgestimmt zur Bestrahlung des menschlichen Körpers einzusetzen. Hierdurch lassen sich insbesondere komplexe Kombinationsbehandlungen durchführen, bei denen beispielsweise die Strahlungsspektren gleichzeitig, überlappend und/oder nacheinander oder gepulst gesteuert werden können, sodass sich simultan unterschiedliche Aspekte der Körperregeneration ansprechen lassen.

Vorzugsweise umfasst die Bestrahlungsquelle zumindest einen vierten LED-Chip, der ein viertes Strahlungsspektrum mit einem von dem ersten Strahlungspeak und dem zweiten Strahlungspeak und dem dritten Strahlungspeak verschiedenen vierten Strahlungspeak aussenden kann, wobei der vierte LED-Chip mit dem zumindest einen ersten LED-Chip und dem zumindest einen zweiten LED-Chip und dem zumindest einen dritten LED-Chip unter der gemeinsamen Linse in einem LED-Gehäuse angeordnet ist und separat von diesen ansteuerbar ist. Die separate Ansteuerbarkeit ermöglicht die individuelle Steuerung aller zumindest einen ersten, zweiten, dritten und vierten LED-Chips, sodass sich auch komplexe Behandlungsmuster mit alternativ wechselnden oder kombiniert aktivierten Strahlungspeaks einstellen lassen.

Vorzugsweise umfasst die Bestrahlungsquelle zumindest einen fünften LED-Chip, der ein fünftes Strahlungsspektrum mit einem von dem ersten Strahlungspeak und dem zweiten Strahlungspeak und dem dritten Strahlungspeak und dem vierten Strahlungspeak verschiedenen fünften Strahlungspeak aussenden kann, wobei der fünfte LED-Chip mit dem zumindest einen ersten LED-Chip und dem zumindest einen zweiten LED-Chip und dem zumindest einen dritten LED-Chip und dem zumindest einen vierten LED-Chip unter der gemeinsamen Linse in einem LED-Gehäuse angeordnet ist und separat von diesen ansteuerbar ist. Die separate Ansteuerbarkeit ermöglicht die individuelle Steuerung aller zumindest einen ersten, zweiten, dritten, vierten und fünften LED-Chips, sodass sich auch komplexe Behandlungsmuster mit alternativ wechselnden oder kombiniert aktivierten Strahlungspeaks einstellen lassen.

Auch wird damit die Körperbestrahlungsvorrichtung vielseitig einsetzbar, da sich auch LED-Chips unter der gemeinsamen Linse befinden können, die nicht in derselben Strahlungsbehandlung eingesetzt werden, es aber ermöglichen, in einem Gerät unterschiedliche Behandlungen durchzuführen (Kombinationsgerät). So kann beispielsweise eine Körperbestrahlungsvorrichtung zwei LED-Chips, die für eine kombinierte Erzeugung von Previtamin D3 eingesetzt werden, und/oder zwei oder mehr LED-Chips, die für die Bräunung eingesetzt werden, und/oder zwei oder mehr LED-Chips, die für die Haut- und Wundregeneration eingesetzt werden, und/oder zwei oder mehr LED-Chips, die für die (kosmetische) Akne-Behandlung eingesetzt werden, unter derselben Linse aufweisen, wodurch vorteilhaft ein Mehrzweckgerät geschaffen ist, das entsprechend günstig in der Herstellung, der Anschaffung und im Betrieb ist.

Es ist möglich, innerhalb der Bestrahlungsquelle auch mehr als einen ersten, zweiten, usw. LED-Chip vorzusehen, beispielsweise zwei LED-Chips mit ersten Strahlungspeak und einem LED-Chip mit zweitem Strahlungspeak. In einer anderen Ausführung kann beispielsweise vorgesehen sein, dass von dem ersten LED-Chip jeweils einer mehr vorgesehen ist als von dem zweiten LED-Chip. In noch einer weiteren Ausgestaltung kann dann vorgesehen sein, dass von dem zweiten LED-Chip jeweils einer mehr vorgesehen ist als von dem dritten LED-Chip, sodass bei der Ausführung insgesamt sechs LEDs vorgesehen sind, dreimal der erste LED-Chip, zweimal der zweite LED-Chips und einmal der dritte LED-Chip. Zweckmäßigerweise sind nicht mehr als sechs LED-Chips unter einer gemeinsamen Linse angeordnet, wobei gleichartige LED-Chips auch gemeinsam ansteuerbar und/oder kontaktierbar sind.

Während sich die Strahlungspeaks der ersten, zweiten, usw. Strahlungspeaks unterscheiden, ist hierdurch nicht ausgeschlossen, dass die zugehörigen Strahlungsspektren sind partiell überlappen. Zweckmäßigerweise ist jedoch die resultierende Intensität und die Strahlungsdichte des Überlappungsbereichs hierbei geringer als jeder der einzelnen Strahlungspeaks.

Die Körperbestrahlungsvorrichtung kann ferner neben den Bestrahlungsquellen mit LED-Chips auch weitere Strahlung erzeugende Mittel aufweisen, beispielsweise Niederdruckröhren oder Hochdruckstrahler.

Es ist möglich, eine Universalbasis vorzusehen, die mit unterschiedlichen LED-Chips bestückt werden kann, wobei die Steuerung je nach bestückten LED-Chip erfolgt.

Zweckmäßigerweise sind die LED-Chips von der eine erste Primäroptik bildenden Linse bedeckt. Die Linse kann hierbei eine konvexe Außenkontur und konkave Innenkontur nach Art einer Sammellinse aufweisen, wobei der Durchmesser der Linse mindestens dreimal, vorzugsweise mindestens viermal so groß ist wie die Diagonale des größten der LED-Chips. Durch den Abstand, den die Linse in der Projektion zu den LED-Chips aufweist, der zweckmäßig zumindest so groß ist wie die Diagonale des entsprechenden Chips, erfolgt eine gute Verteilung der Strahlung der LED-Chips auch dann, wenn diese nicht zentrisch unter der Linse angeordnet sind.

Gemäß einer günstigen Ausgestaltung ist vorgesehen, dass zwischen den LED-Chips und der Linse eine Filterlage angeordnet ist. Die Filterlage kann als Filterglas mit Kurz- und/oder Langpassfilter ausgeführt sein und dient insbesondere dazu, Strahlung mit kritischem Strahlungswellenlängen unterhalb und/oder oberhalb einer als schwellenwert festgelegten Wellenlänge herauszufiltern. Solche Filterlagen sind bei breiten Strahlungsspektren von Hoch- und/oder Niederdrucklampen zweckmäßig, bei LED-Chips mit entsprechend schmalen Strahlungsspektren unter Umständen verzichtbar - bei relativ kleinen Wellenlängen und/oder bei nahe beieinander liegenden Peaks ist ein Filter besonders zweckmäßig.

Gemäß einer anderen günstigen Ausgestaltung ist vorgesehen, dass die Linse eine integrierte Filteranordnung aufweist, die unerwünschte Strahlungsspektren am Durchtritt hindert und damit eliminiert. Bei der Filteranordnung handelt es sich zweckmäßig um einen Filter mit Kurz- und/oder Langpassfilter, der Strahlung, die außerhalb eines gewünschten Spektrums liegt, am Durchtritt hindert und damit eliminiert.

Bevorzugt ist jedoch eine Ausgestaltung, bei die Strahlungsspektren der LED-Chips derart begrenzt sind, dass ein Filter nicht benötigt wird. Hierdurch wird vorteilhaft auch eine günstige Temperaturentwicklung der Bestrahlungsquelle erreicht, denn die herausgefilterten Wellen bewirken sonst eine Erhöhung der Temperatur und damit einen erhöhten Kühlaufwand und einen schlechteren Wirkungsgrad.

Ein besonders günstiges Bestrahlungsergebnis ergibt sich dann, wenn die Strahlungspeaks der LED-Chips durch eine Wellenlänge definiert sind, und wenn das Spektrum der LED-Chips zu mehr als zwei Dritteln, vorzugsweise zu mehr als drei Vierteln, besonders bevorzugt zu mehr als neun Zehnteln innerhalb einer Bandbreite, errechenbar durch das Produkt der Wellenlänge (in nm) und einem Faktor F (also Wellenlänge x F) ist, wobei der Faktor F so ausgewählt ist, dass er zwischen 1 +/- 0,005 und 1 +/- 0,05 liegt, und so den Strahlungspeak von beiden Seiten umschließt. Hierdurch ist das Strahlungsspektrum im Wesentlichen innerhalb einer etwa beiderseits des Peaks gleich großen Breite konzentriert, sodass eine besonders effektive Behandlung des menschlichen Körpers mit definiertem Strahlungspeak erreicht wird. Zugleich ist er durch die ausgesprochen schmale Abweichung um den Strahlungspeak herum sichergestellt, dass schädliche Strahlung allenfalls in untergeordneter Menge ausgesendet wird. Besonders bevorzugt liegt sogar ein Anteil von 99%, vorzugsweise 99,9% und besonders bevorzugt von 99,99% innerhalb der genannten Grenze.

Bevorzugt ist Strahlungspeak der LED-Chips, also beispielsweise des ersten, des zweiten, des dritten, des vierten, des fünften LED-Chip ausgewählt aus der Gruppe umfassend 290 nm +/- 2 nm, 297 nm +/- 2 nm, 310 nm +/- 5 nm, 365 nm +/- 10 nm, 620 nm +/- 15 nm, 660 nm +/- 15 nm, 465 nm +/- 20 nm, 740 nm +/- 20 nm und 840 nm +/- 20 nm; und. Hierbei lassen sich bestimmte Wellenlängen zu therapeutisch und/oder kosmetisch günstigen Kombinationen mit aufeinander abgestimmten Strahlungsintensitäten zusammensetzen, wodurch physiologische Effekte in dem zu bestrahlenden Körper erreicht werden. Durch die Auswahl bestimmter Strahlungspeaks wird die Bestrahlung und damit mögliche Schädigung oder Beanspruchung des menschlichen Körpers mit nicht oder nicht optimal wirksamer Strahlung reduziert und entsprechend geschont. Die jeweils angegebenen Toleranzen bringen zum Ausdruck, dass der Strahlungspeak des jeweiligen LED-Chips nicht exakt und stets identisch reproduzierbar ist, sondern in engen Grenzen variieren kann.

Die vorgenannten Strahlungspeaks gehören zur Gruppe der kosmetisch-hygienisch nützlichen Strahlung, und zwar unbeschadet einer möglicherweise zugleich existierenden medizinischen Wirkung. Insbesondere erzeugt die kosmetisch-hygienisch nützliche Strahlung eine photobiologische Wirkung bei einer bestrahlten Person. Die kosmetisch-hygienisch nützliche Strahlung trifft hierbei auf die Haut der Person auf, kann aber je nach konkreter Wellenlänge in tiefere Regionen des Körpers eindringen. Die Wirkung umfasst beispielsweise eine Bräunung der Haut, aber auch weitere physiologische und psychologische Effekte resultieren aus der Bestrahlung. Kosmetisch-hygienisch nützliche e Strahlung umfasst das Spektrum der ultravioletten (UV-) Strahlung außerhalb des UV-C-Spektrums, der sichtbaren (VIS-) Strahlung und der nahe Infrarot (nIR-) Strahlung. Die UV-Strahlung außerhalb des UV-C-Spektrums weist hierbei Wellenlängen im Spektrum zwischen 280 nm und ca. 380 nm auf, die VIS-Strahlung weist hierbei Wellenlängen im Spektrum zwischen ca. 380 nm und ca. 780 nm auf, die nIR-Strahlung weist hierbei Wellenlängen im Spektrum zwischen ca. 780 nm und 1400 nm auf. Die genannten Spektren gehen ineinander über. Je nach kosmetischer oder hygienischer Anwendung kann die Bestrahlung auf ein Teilspektrum der genannten Spektren konzentriert sein. Hierzu können kosmetisch-hygienisch nützliche Strahlung emittierende Anordnungen auch dediziert einzelnen Wellenlängen zugeordnet sein, z.B. der UV-Strahlung. Man erkennt, dass die vorstehend konkreter angeführten bevorzugten Strahlungspeaks der LED-Chips alle in den Bereich der kosmetisch-hygienisch nützliche Strahlung fallen. Die Aufzählung der konkret genannten Strahlungspeaks der LED-Chips und/oder deren beliebige Kombinationen aus zwei oder mehr Strahlungspeaks, die hiermit als Teil der Offenbarung ausdrücklich genannt werden, ist jedoch nicht abschließend.

Ein interessanter Aspekt ist hierbei ferner, dass die Kombinationsbehandlung nicht nur das Aussetzen des menschlichen oder tierischen Körpers der Strahlung mit den entsprechenden Wellenlängen ermöglicht, sondern darüber hinaus das gezielte in Beziehung setzen dieser Strahlungspeaks. So kann sowohl die Intensität der Strahlung im Verhältnis zueinander eingestellt werden als auch die Dauer und/oder die Reihenfolge der Bestrahlungen.

Bevorzugt ist vorgesehen, dass zumindest ein Strahlungspeak, der von einem der LED-Chips ausgesendet wird, zum Beispiel derjenige des ersten LED-Chips oder eines weiteren LED-Chips, eine Wellenlänge aufweist, die außerhalb des Spektrums des sichtbaren Lichts liegt. Im Unterschied zu Körperbestrahlungsvorrichtungen, die ausschließlich ein breites Spektrum sichtbaren Lichts aussenden, wird hierdurch eine Körperbestrahlungsvorrichtung bereitgestellt, die auch zumindest einen Strahlungspeak mit einer Wellenlänge im Bereich des Spektrums der ultravioletten (UV-) Strahlung außerhalb des UV-C-Spektrums und/oder der nahe Infrarot (nIR-) Strahlung aufweist. Es ist möglich, dass weitere Strahlungspeaks eine Wellenlänge im Bereich des Spektrums der sichtbaren (VIS-) Strahlung und/oder außerhalb derselben aufweisen. Hierdurch lassen sich günstig auch Bestrahlungsmodi einstellen, die Strahlung im nicht sichtbaren Bereich bereitstellen.

Vorzugsweise weist der zumindest eine Strahlungspeak, der außerhalb des Spektrums des sichtbaren Lichts liegt, eine Wellenlänge kleiner als 380 nm auf und liegt damit im Bereich des Spektrums der ultravioletten (UV-) Strahlung außerhalb des UV-C-Spektrums. Es ist möglich, mehrere Strahlungspeaks aus diesem Spektrum vorzusehen. Der zumindest eine Strahlungspeak, der außerhalb des Spektrums des sichtbaren Lichts liegt, weist beispielsweise eine Wellenlänge auf, die ausgewählt ist aus der Gruppe umfassend 290 nm +/-2 nm; 297 nm +/- 2 nm; 310 nm +/- 5 nm; 365 nm +/- 10 nm.

Der zumindest eine Strahlungspeak, der außerhalb des Spektrums des sichtbaren Lichts liegt, weist alternativ eine Wellenlänge größer als 780 nm auf und liegt damit im Bereich des Spektrums der nahe Infrarot (nIR-) Strahlung. Es ist möglich, mehrere Strahlungspeaks aus diesem Spektrum vorzusehen. Der zumindest eine Strahlungspeak, der außerhalb des Spektrums des sichtbaren Lichts liegt, weist beispielsweise eine Wellenlänge auf, die ausgewählt ist aus der Gruppe umfassend 840 nm +/- 20 nm.

Die vorgenannten Strahlungspeaks, die außerhalb des Spektrums des sichtbaren Lichts liegen, können auch mit weiteren Strahlungspeaks kombiniert werden, die von anderen LED-Chips ausgesendet werden, die im Spektrum des sichtbaren Lichts enthalten sind.

Vorzugsweise ist der Linse, die eine Primäroptik bildet, ein Kollimations-Reflektor zum Kollimieren der Strahlung nachgeordnet, der die Strahlung aller LED-Chips gleichmäßig kollimiert und damit eine Sekundäroptik bildet. Der Kollimations-Reflektor ist hierzu vorzugsweise nach Art eines Kegelstumpfes oder eines endseitig abgeschnittenen Rotationsparaboloides mit stark reflektierendem Innenumfang beispielsweise aus Aluminium ausgebildet, wodurch die durch die Linse austretende Strahlung homogenisiert wird und insbesondere im Bereich der angestrahlten Fläche keine relativen Intensitätspeaks entstehen. Besonders überraschend ist hierbei, dass auch bei Wellenlängenbereichen, die sich um bis zu dem Faktor zwei unterscheiden, derselbe Kollimations-Reflektor eingesetzt werden kann, und gleichwohl die ausgesendete Strahlung nahezu homogen auf den menschlichen Körper auftrifft. Zur Regelung der Auftreffintensitäten können die LED-Chips entsprechend angesteuert werden, was sich durch Einmessen der Körperbestrahlungsvorrichtung leicht erreichen lässt.

Zweckmäßig weist der Kollimations-Reflektor eine kleine Öffnung auf der der Linse zugekehrten Seite und eine größere Öffnung auf der der Linse abgekehrten Seite auf, die zweckmäßig kreisförmig ist. Die die Linse passierende Strahlung tritt durch die kleine Öffnung ein und kollimierte Strahlung tritt durch die große Öffnung aus. Damit ist der Kollimations-Reflektor dem LED-Gehäuse und der Linse vollständig vorgelagert angeordnet.

Gemäß einem günstigen Aspekt ist vorgesehen, dass mehr als die Hälfte der LED-Chips exzentrisch zu einem durch die Projektion des Scheitels der Linse auf die Basis definierten Mittelpunkt angeordnet sind. Überraschend hat sich herausgestellt, dass sich zumindest zwei LED-Chips mit einer gleichmäßigen Abstrahlung besonders günstig dann auf der Basis anordnen lassen, wenn weder der erste LED-Chip noch der zweite LED-Chip exakt auf den Mittelpunkt angeordnet ist, sondern jeweils vom Mittelpunkt beabstandet angeordnet ist. Zwar führt dies zunächst zu einer weniger homogenen Ausleuchtung, die jedoch insbesondere durch nachgeschaltete Kollimations-Reflektoren nahezu vollständig kompensiert wird, während das Anordnen eines der LED-Chips auf den Mittelpunkt von dem nachgeschalteten Kollimations-Reflektor schlechter homogenisiert wird.

Vorzugsweise ist bei mehr als zwei LED-Chips einer der LED-Chips auf einem durch die Projektion des Scheitels der Linse auf die Basis definierten Mittelpunkt angeordnet, wobei hierbei vorzugsweise derjenige Chip ausgewählt wird, der eine geringe Strahlungsintensität aufweist und dementsprechend einen hohen Energieverbrauch, wenn die Intensität erhöht werden soll.

Zweckmäßigerweise ist jedoch vorgesehen, dass alle LED-Chips exzentrisch zu einem durch die Projektion des Scheitels der Linse auf die Basis definierten Mittelpunkt angeordnet sind, wodurch sich bei allen LED-Chips und ihren respektiven Strahlungsspektren eine insbesondere durch den nachgeschalteten Kollimations-Reflektor einstellende gleichmäßige Ausleuchtung erreichen lässt.

Gemäß einer bevorzugten Ausgestaltung ist vorgesehen, dass zumindest einer der LED-Chips Strahlung gepulst aussenden kann. Die gepulste Strahlung kann je nach Wellenlängen, vorzugsweise bei Wellenlängen bei mehr als 280 nm, bevorzugt bei mehr als 700 nm, günstige Effekte insbesondere bei der Behandlung des menschlichen Körpers erzielen und hierbei besonders anregend wirken. Es ist möglich, das Pulsen des einen LED-Chips mit dem Pulsen des anderen LED-Chips simultan oder abwechselnd vorzusehen. Zweckmäßigerweise werden die gepulsten LED-Chips im Bereich des sichtbaren Lichts sowie des nahen Infrarotspektrums eingesetzt. Es ist aber auch möglich, die LED-Chips im Bereich des UV-A und des UV-B Spektrums zu pulsen.

Beim Pulsen der LED-Chips lässt sich in einer ersten vorteilhaften Ausführung der Parameter Pulsweitenverhältnis, also das Verhältnis an/aus bzw. der Dauer des Aussendens von Strahlung und der Dauer des Nicht-Aussendens von Strahlung in weiten Grenzen einstellen. In einer ersten bevorzugten Ausführung ist die An-Dauer gleich der Aus-Dauer. In einer zweiten bevorzugten Ausführung ist die An-Dauer zwischen 25% und 200% länger als die Aus-Dauer. Durch das Pulsen der LED-Chips in den vorgenannten Pulsweitenverhältnissen scheint überraschend ein signifikant besserer Effekt der Bestrahlung erreicht zu werden, als wenn eine kontinuierliche Bestrahlung erfolgt.

Beim Pulsen der LED-Chips lässt sich in einer zweiten vorteilhaften Ausführung, die wahlweise zugleich mit der ersten Ausführung verwirklicht sein kann, der Parameter Frequenz, also die Anzahl der an/aus Pulse je Zeiteinheit, in weiten Grenzen einstellen. Eine besonders effektive Frequenz wird ausgewählt zwischen 0,25 Hz und 500 Hz, bevorzugt zwischen 1 Hz und 100 Hz, besonders bevorzugt zwischen 8 Hz und 15 Hz, vorzugsweise bei 10 Hz. Eine weitere günstige Frequenz liegt in dem Frequenzband, das größer oder gleich derjenigen Frequenz ist, das vom menschlichen Auge als intermittierend erfasst wird, also z.B. oberhalb von 50 Hz. Hier kommt z.B. eine Frequenz von 60 Hz, 120 Hz oder 240 Hz in Betracht.

Das separate Pulsen der ersten LED-Chips, zweiten LED-Chips, usw. unabhängig voneinander, wenn auch möglicherweise abgestimmt aufeinander, ist auch durch die separate Ansteuerbarkeit der LED-Chips verwirklicht, ohne dass diese weiteren Merkmale in jeder Ausführung bereitgestellt sein müssen.

Zweckmäßigerweise sind auf dem Träger eine Mehrzahl von Linsen angeordnet, die jeweils mehrere LED-Chips in jeweils einem LED-Gehäuse bedecken. Gemäß einer bevorzugten Weiterbildung sind alle auf dem Träger so gebildeten Strahlungsquellen gleichartig ausgebildet, sodass jede Linse dieselbe Anzahl mit den gleichartigen LED-Chips bedeckt. Es ist aber möglich, die auf einer Bestrahlungsanordnung vorgesehenen Bestrahlungsquellen auch verschiedenartig auszubilden.

Gemäß einer günstigen Weiterbildung ist vorgesehen, dass die Körperbestrahlungsvorrichtung ferner einen Träger umfasst, auf dem eine Mehrzahl von Bestrahlungsquellen angeordnet sind, die jeweils mehrere LED-Chips unter einer gemeinsamen Linse aufweisen. Hierbei ist zweckmäßig vorgesehen, dass die gleichen LED-Chips auf dem Träger einheitlich angesteuert werden.

Eine günstige Ausgestaltung sieht hierbei vorteilhaft vor, dass auf dem Träger zwanzig Bestrahlungsquellen oder ganzzahliges Vielfaches davon angeordnet sind. Hierdurch kann ein Träger mit jeweils zwanzig Bestrahlungsquellen mit mehreren LED-Chips ausgestattet werden, die sich modulartig innerhalb einer Körperbestrahlungsvorrichtung anordnen und betreiben und sich trotzdem leicht austauschen lassen.

Vorteilhaft ist vorgesehen, dass die Basis der Bestrahlungsquellen jeweils auf dem Träger angeordnet ist. Die Basis kann hierbei beispielsweise einstückig auf dem Träger ausgespart sein oder aber auf den Träger aufgeklebt, geklemmt oder angeordnet sein, z.B. in einer hierfür vorgesehenen Aussparung. Vorzugsweise ist die Basis der Grund des LED-Gehäuses, das überdies eine ringförmige Wandung aufweist und damit eine Art Kammer oder Aufnahme ausbildet. Einer der Basis abgekehrte Unterseite ist vorzugsweise flach und kann auf einer planen Seite des Trägers festgelegt werden.

Gemäß einer anderen günstigen Ausführung ist vorgesehen, dass alle Bestrahlungsquellen auf dem Träger gleich ausgebildet sind, sodass die jeweiligen LED-Chips gemeinsam, parallel oder in Serie, angesteuert werden können. So werden die ersten LED-Chips gemeinsam angesteuert, die zweiten LED-Chips gemeinsam angesteuert, usw. Alternativ können die Bestrahlungsquellen auf dem Träger verschiedenen ausgebildet sein; in diesem Fall müssen die LED-Chips entsprechend individuell angesteuert werden. Auch Kombinationen aus beiden Ausführungen sind möglich, also beispielsweise ein Feld (Array) gleich ausgebildeter erster Bestrahlungsquellen und zusätzlich einzelne hiervon abweichend ausgebildete zweite Bestrahlungsquellen.

Der Bestrahlungsraum der Körperbestrahlungsvorrichtung, in dem der zu bestrahlende menschliche Körper nach Art eines Tunnels umfasst ist, kann entweder als vertikal ausgerichteter Tunnel mit einem beweglichen Wandelement oder als Liege mit einem aufschwenkbaren Abdeckelement ausgebildet sein kann. Es ist möglich, den Bestrahlungstunnel zur Abfuhr von Wärme mit einem Luftstrom zu beaufschlagen, der den Innenraum des Tunnels kühlt.

Eine günstige Ausführungsform sieht vor, dass der der erste Strahlungspeak 290 nm +/- 2 nm und der zweite Strahlungspeak 297 nm +/- 2 nm beträgt. Mit dieser Kombination innerhalb des UV-B Spektrums lässt sich günstig das Previtamin D3 erzeugen, wobei zugleich eine Schädigung der DNA der behandelten Person besonders gering ist.

Eine andere günstige Ausführungsform sieht vor, dass der erste Strahlungspeak 310 nm +/- 5 nm und der zweite Strahlungspeak 365 nm +/-10 nm beträgt. Mit dieser Kombination einer Wellenlänge aus dem UV-B Spektrum und einer Wellenlänge aus dem UV-A Spektrum lässt sich günstig die Bräunung der menschlichen Haut durch Pigmentverfärbung erreichen.

Eine weitere günstige Ausführungsform sieht vor, dass der erste Strahlungspeak 620 nm +/- 15 nm und der zweite Strahlungspeak 660 nm +/-15 nm beträgt. Mit dieser Kombination einer Wellenlänge aus dem sichtbaren (VIS) Spektrum lassen sich kosmetische Effekte, Hautverjüngung und/oder Wundheilung erreichen und/oder fördern.

In vorteilhafter Weiterbildung ist dann vorgesehen, dass der der dritte Strahlungspeak 740 nm +/- 20 nm und der vierte Strahlungspeak 840 nm +/-20 nm beträgt. Hierdurch wird überdies ein Wellness-Effekt und die Regeneration von Sehnen, Faszien und der Muskulatur gefördert und/oder erreicht.

In weiterer Weiterbildung ist dann vorgesehen, dass der fünfte Strahlungspeak 465 nm +/- 20 nm beträgt. Hierdurch wird auch Akne der bestrahlten Haut reduziert.

Noch eine weitere günstige Ausführungsform sieht vor, dass der erste Strahlungspeak 465 nm +/- 20 nm und der zweite Strahlungspeak 660 nm +/-20 nm und der dritte Strahlungspeak 840 nm +/- 20 nm beträgt. Hierdurch wird insbesondere Akne der Haut vorteilhaft zurückentwickelt.

Gemäß einem Aspekt wird die Verwendung der Körperbestrahlungsvorrichtung wie vorstehend beschrieben zur kosmetischen, medizinischen, psychologischen, Wellness- und Regenerationsbehandlung angegeben. Insbesondere kann die Körperbestrahlungsvorrichtung zur nicht-therapeutischen Behandlung des menschlichen und/oder des tierischen Körpers vorgesehen sein, beispielsweise zur psychologischen Behandlung oder zur kosmetischen Behandlung.

Gemäß einem Aspekt ist ein Verfahren zum nicht-therapeutischen Bestrahlen einer Person mit kosmetisch-hygienisch nützlichen Strahlung angegeben, umfassend eine Bestrahlungsquelle mit einer Basis und mit zumindest einen ersten LED-Chip, der ein Strahlungsspektrum mit einem ersten Strahlungspeak aussenden kann, und mit zumindest einem zweiten LED-Chip, der ein zweites Strahlungsspektrum mit einem von dem ersten Strahlungspeak verschiedenen zweiten Strahlungspeaks aussenden kann, wobei sich das Verfahren dadurch auszeichnet, dass der erste LED-Chip und zweite LED-Chip unter einer gemeinsamen Linse angeordnet sind und separat angesteuert werden. Hierdurch ist es vorteilhaft möglich, die Strahlungsspektren des ersten LED-Chips und des zweiten LED-Chips hinsichtlich ihrer Intensität aufeinander abzustimmen und ist zugleich sichergestellt, dass die von der Bestrahlungsquelle ausgesandte Strahlung homogen auf die zu bestrahlende Person auftrifft. Für das Verfahren kann die vorstehend beschriebene Körperbestrahlungsvorrichtung einschließlich all ihrer optionalen Weiterbildungen vorgesehen werden. Vorzugsweise sind entsprechend der vorstehenden Beschreibung auch dritte, vierte und fünfte LED-Chips möglich, die unter der gemeinsamen Linse angeordnet sind und separat angesteuert werden. Das Verfahren sieht vorteilhaft die Bestrahlung eines menschlichen Körpers einer Person oder eines Teils eines menschlichen Körpers einer Person vor. Hierdurch unterscheidet es sich von Bestrahlungsverfahren, die Strahlung auf Gegenstände richten und bei denen keine gesundheitlichen Einschränkungen hinsichtlich Dosis und Strahlungsspektrum zu beachten sind. Durch das Unterbringen von mehreren LED-Chips in einem LED-Gehäuse ist es möglich, die Körperbestrahlungsvorrichtung nahe zu dem zu bestrahlenden Körper anzuordnen und trotzdem eine homogene Beaufschlagung mit allen Strahlungspeaks auf engem Raum zu erreichen. Durch die körpernahe Anordnung der Körperbestrahlungsvorrichtung wirkt die Strahlung intensiver und die Behandlungszeit ist vorteilhaft verkürzt.

Weitere Vorteile, Weiterbildungen und Eigenschaften der Erfindung ergeben sich aus der nachfolgenden Beschreibung von bevorzugten Ausführungsbeispielen sowie aus den abhängigen Ansprüchen. Hier beschriebene medizinische, chirurgische oder therapeutische Methoden, dienen einer besseren Veranschaulichung und sind nicht Teil der Erfindung.

Die Erfindung wird nunmehr unter Bezugnahme auf die anliegenden Zeichnungen anhand von bevorzugten Ausführungsbeispielen näher erläutert.
- Fig. 1: zeigt eine schematische Seitenansicht eines bevorzugten Ausführungsbeispiels einer erfindungsgenmäßen Körperbestrahlungsvorrichtung.
- Fig. 2: zeigt eine explodierte Darstellung eines Bestrahlungsmoduls, das in der Körperbestrahlungsvorrichtung aus Fig. 1 eingebaut ist.
- Fig. 3A: zeigt einen Querschnitt durch eine Bestrahlungsquelle aus dem Bestrahlungsmodul aus Fig. 2.
- Fig. 3B: zeigt eine Draufsicht auf die Bestrahlungsquelle aus Fig. 3A
- Fig. 4A: zeigt einen Querschnitt durch eine andere Bestrahlungsquelle aus dem Bestrahlungsmodul aus Fig. 2.
- Fig. 4B: zeigt eine Draufsicht auf die Bestrahlungsquelle aus Fig. 4A
- Fig. 5: zeigt die Intensität der Strahlung von zwei LED-Chips bei einer Bestrahlungsquelle gemäß Beispiel 1.

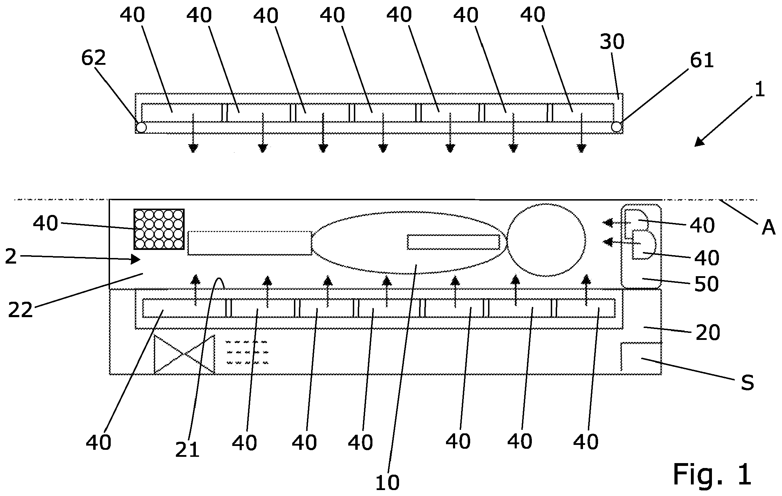

Fig. 1 zeigt eine Körperbestrahlungsvorrichtung 1 zum Bestrahlen einer mit ihrem menschlichen Körper 10 schematisch dargestellten Person mit kosmetisch-hygienisch nützlicher Strahlung, die ein unteres Gehäuseteil 20 und ein oberes Gehäuseteil 30 umfasst, die entlang einer Achse A gelenkig miteinander verbunden sind. Das obere Gehäuseteil 30 kann nach oben geschwenkt werden, um den Zugang für den Benutzer 10 frei zu geben, und kann herabgeschwenkt werden, damit die Gehäuseteile 20, 30 eine tunnelartige Röhre 2 umschließen, in der der Benutzer 10 liegt.

Die Gehäuseteile 20, 30 sind in Acrylglas gekapselt, wobei bei dem unteren Gehäuseteil 20 eine Liegefläche 21 aus Acrylglas ausgebildet ist. Es ist möglich, die Liegefläche mit einer Silikonmatte, die an die Liegefläche 10 angeschlossen ist, auszustatten, die nachgiebig ist und für die Person 10 eine angenehmere Haptik bereitstellt. Das Acrylglas und die Silikonmatte sind jeweils für zumindest Teile der kosmetisch-hygienisch nützlichen Strahlung durchlässig.

In dem unteren Gehäuseteil 20 und dem oberen Gehäuseteil 30 sind jeweils Bestrahlungsmodule 40 angeordnet, die in den Gehäuseteilen 20, 30 in Richtung auf die Röhre 2 gerichtet sind. Die Bestrahlungsmodule 40 sind rechteckig aufgebaut und in dem unteren Gehäuseteil 20 nebeneinander liegend parallel zu der Liegefläche 21 angeordnet. Weiterhin sind an dem zu der Liegefläche 21 in etwa senkrechten vertikalen Abschnitt 22 des unteren Gehäuseteil 20 ebenfalls Bestrahlungsmodule 40 vorgesehen, die die Person 10 bestrahlen können. In dem oberen Gehäuseteil 30 sind in einer Reihe eine Mehrzahl von Bestrahlungsmodulen 40 jeweils aneinander stoßend angeordnet, wobei die in einer Reihe angeordneten Bestrahlungsmodule gegenüber der benachbarten Reihe um einen Winkel abgewinkelt sind, um die halbrunde Kontur des oberen Gehäuseteils 30 innerhalb des Gehäuseteils 30 nachvollziehen zu können. Der Winkel beträgt je nach Radius und je nach Größe der Bestrahlungsmodule zwischen 5° und 25°, vorzugsweise etwa 10°.

An einem Kopfende der Röhre 2 ist eine Schulterbräuneinrichtung 50 angeordnet, die insbesondere den Kopf und die Schulter des Benutzers 10 bestrahlt, wobei innerhalb der Schulterbräuneinrichtung 50 zwei weitere Bestrahlungsmodule 40 angeordnet sind.

Die Bestrahlungsmodule 40 sind mit einer Steuerung S der Vorrichtung 1 verbunden.

Die Körperbestrahlungsvorrichtung 1 weist überdies eine Reihe von weiteren Komponenten auf, die das Bestrahlungserlebnis verbessern. Als erstes Interface für die Kommunikation mit der Person 10 ist an einer Innenseite des oberen Gehäuseteils 30 ein Flachbildschirm mit Touch-Sensitivität vorgesehen, der Eingaben der Person 10 erlaubt und ferner das Abspielen eines Unterhaltungsprogramms ermöglicht. Ferner sind im Kopfbereich der Person 10 Lautsprecher angeordnet, die eine akustische, z.B. musikalische Untermalung des Bestrahlungserlebnis, gegebenenfalls im Zusammenwirken mit dem visuellen Unterhaltungsprogramm ermöglichen. An einer Außenseite des oberen Gehäuseteils 30 ist ein zweites Interface für die Kommunikation mit der Person 10 vorgesehen, über das Programme ausgewählt werden können. Als drittes Interface für die Kommunikation mit der Person 10 ist eine Sprachsteuerung vorgesehen, die über einen an zumindest einem der Gehäuseteile angebrachten Lautsprecher Spracheingaben erfasst, rechnergestützt auswertet und in Steuerbefehle für die Körperbestrahlungsvorrichtung 1 umsetzt. Ferner weist die Körperbestrahlungsvorrichtung 1 eine Belüftung der Röhre 2 auf, die frische Luft zuführt und erwärmte Luft in der Röhre abführt. Weiterhin weist die Körperbestrahlungsvorrichtung 1 eine Beduftungseinrichtung auf, die es ermöglicht, die frische Luft mit einem aus einer Mehrzahl von Düften zu parfümieren. Schließlich umfasst die Körperbestrahlungsvorrichtung 1 auch alle erforderlichen elektrischen und elektronischen Komponenten zum Betrieb

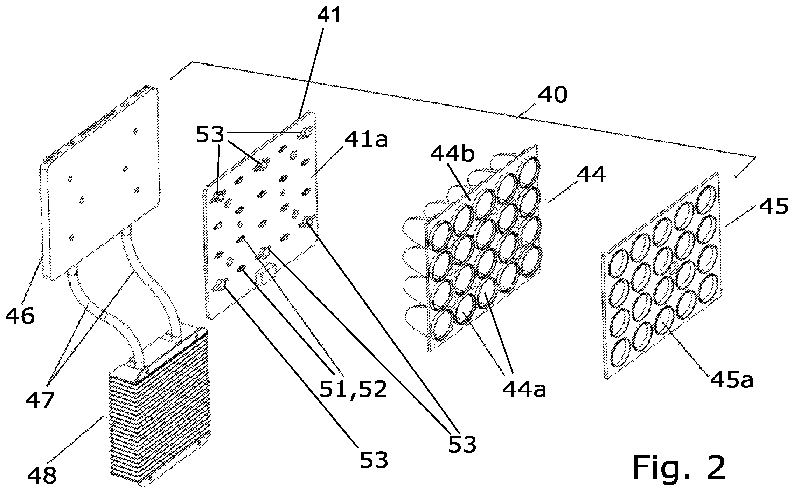

In Fig. 2 ist eine Explosionsdarstellung eines Bestrahlungsmoduls 40 veranschaulicht. Man erkennt, dass auf jeweils einer Fläche 41a eines Träger 41 eine Mehrzahl von insgesamt zwanzig Bestrahlungsquellen 50 angebracht sind, die jeweils zumindest einen ersten LED-Chip 51 und einen zweiten LED-Chip 52 aufweisen, die über den Träger 41 mit einer elektrischen Energieversorgung kontaktiert sind. Es ist möglich, die LED-Chips auch in einer anderen Zahl und/oder Anordnung als wie vorliegend in 4x5 Feldanordnung vorzusehen.

Man erkennt in Fig. 2, dass auf insgesamt sechs LEDs 51, 52 eine gemeinsame Linse 53 auf den Träger 41 dargestellt ist, die an der Fläche 41a festgelegt ist und die eine nach außen gewölbte Form aufweist und die für die Strahlung der LED-Chips 51, 52 durchlässig ist.

Dem Träger 41 mit den LEDs 42, 43 vorgelagert ist eine Baueinheit 44 mit 20 identisch ausgebildeten Kollimations-Reflektoren 44a vorgesehen, wobei das Lochmaß der Kollimations-Reflektoren 44a auf die Linsen 53 abgestimmt ist. Die Reflektoren 44a sind hierzu in einem von den LEDs 42, 43 beabstandeten Bereich mit einer Scheibe 44b, die Durchbrechungen für die Kollimations-Reflektoren 44a aufweist, verbunden, sodass die Baueinheit 44 wie ein Teil gehandhabt werden kann.

Der Baueinheit 44 vorgelagert ist eine Ringscheibe 45, die eine der Anzahl der Kollimations-Reflektoren 44a entsprechende Anzahl von kreisförmigen Ausnehmungen 45a in einem Plattenkörper aufweist, die in ihrem Innenumfang mit einem fluoreszierenden Material bestrichen sind. Werden die LEDs 51, 52 zum Emittieren der Strahlung veranlasst, regt diese Strahlung das fluoreszierende Material der Ringe 45a an, und auf Grund des im sichtbaren Bereich stattfindenden Leuchtens der Ringe 45a kann erkannt werden, dass die LEDs 51, 52 auch Strahlung emittieren.

An der den LEDs 51, 52 abgekehrten Seite des Trägers 41 ist eine Wärmeübergangsplatte 46 angeordnet, die als Plattenkörper ausgebildet ist und zur Ableitung der beim Betrieb des LEDs 42, 43 entstehenden Wärme bestimmt ist. Hierzu ist die Wärmeübergangsplatte 46 über eine erste Kühlleitung 47 und eine zweite Kühlleitung 47 mit einem als Wärmetauscher ausgebildeten Kühlkörper 48 verbunden, wobei zwischen der mit Hohlräumen ausgebildeten Wärmeübergangsplatte 46, der ersten Kühlleitung 47, den Kühlkörper 48 und der zweiten Kühlleitung 47 ein zirkulierendes Kühlfluid vorgesehen ist. Die Kühlung der Wärmeübergangsplatte 46 kann insbesondere durch Phasenumwandlung des Kühlfluids zwischen der Wärmeübergangsplatte 46 einerseits und dem Kühlkörper 48 andererseits bewirkt werden.

Die in dem Gehäuseteil 20 bzw. Gehäuseteil 30 verbauten Bestrahlungsmodule 40 sind alle gleichartig aufgebaut, es versteht sich aber, dass die Bestrahlungsmodule auch in Abhängigkeit von der Lichtempfindlichkeit bestimmter Partien des Benutzers 10 unterschiedlich aufgebaut und/oder angesteuert sein können.

In dem oberen Gehäuseteil 30 ist ein erster Sensor 61 vorgesehen, der die Eigenschaften des Körpers des Benutzers 10, insbesondere dessen Höhe, Breite, Umfang sowie die Lage der Arme, Beine ermittelt. Je nach erfassten Eigenschaften des Körpers werden die Bestrahlungsmodule 40 in ihrer Strahlung eingestellt. So kann beispielsweise das Bestrahlungsmodul 40, das dem Kopfende abgekehrt ist, ganz abgeschaltet sein, wenn die Beine des Benutzers dieses Bestrahlungsmodul 40 nicht mehr überdecken.

Der Sensor 61 kann alternativ oder zusätzlich bestimmte Hautmerkmale des Körpers des Benutzers 10 erfassen, beispielsweise das Vorliegen von Tätowierungen, von Verbrennungen, von Wunden, von Muttermalen, von Narben, von weißen Flecken, von Pigmentstörungen, der aktuelle Bräunung und auch des Hauttyps. Dieser zweite Sensor, der auch als Kamera ausgebildet sein kann und an den eine Auswertelogik angeschlossen ist, erfasst Färbung und Kontraste der Haut mit einer großen Auflösung und wertet die erfassten Bilder aus, um die genannten Besonderheiten des Körpers zu bestimmen. In Abhängigkeit von den Hautmerkmalen wird dann das Bestrahlungsmodul 40 mit einer reduzierten Leistung betrieben, wenn auf Grund der detektierten Hautmerkmale die Verbrennung der Haut bei normaler Strahlung und Exposition zu befürchten ist.

Schließlich ist in dem oberem Gehäuseteil 30 noch ein zweiter Sensor 62 angeordnet, der die Strahlung der Bestrahlungsmodule 40 bzw. der zugehörigen LEDs 51, 52 detektiert. Der zweite Sensor 62 bzw. dessen Auswerteeinheit vergleicht die erfasste Strahlung mit z.B. in der Steuerung S gespeicherten Soll-Werten, und in Reaktion auf eine Abweichung der detektierten Werte von den Soll-Werten bewirkt die Steuerung, dass die Betriebsparameter der Bestrahlungsmodule 40 so verstellt werden, dass sich ein Einregeln auf den Soll-Wert ergibt.

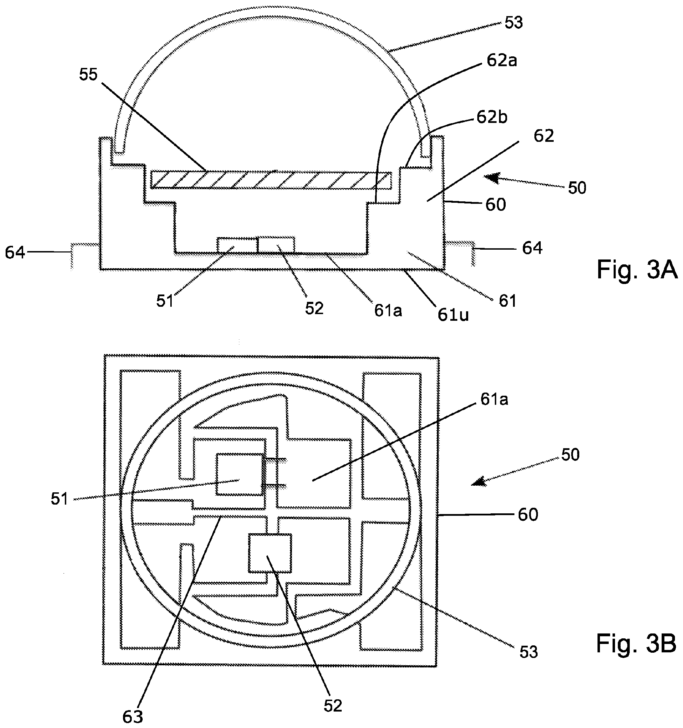

Fig. 3A und 3B zeigen einen Querschnitt und eine Draufsicht auf eine erste Ausführungsform der Bestrahlungsquelle 50, die schematisch und nicht maßstäblich den Aufbau der Bestrahlungsquelle 50 erläutern. Die Bestrahlungsquelle 50 ist im einem LED-Gehäuse 60 aus einem elektrischen Isolator wie Kunststoff oder Keramik untergebracht, das einen rechteckigen, vorliegend quadratischen, Grundriss aufweist und das eine Basis 61 aufweist, deren Unterseite 61u wiederum auf einer Fläche des Trägers 41, beispielsweise eine gut wärmeleitende Platte aus Aluminium, angeschlossen, z.B. angeklebt werden kann. Alternativ kann das LED-Gehäuse 60 auch auf einen Sockel gesteckt werden, der auf dem Träger 41 angebracht ist. Ferner sind elektrische Leitungen auf dem Träger 41 verlegt. Typischerweise weist der Träger 41 etwa zwanzig, in einem Array von 5 x 4 angeordnete LED-Gehäuse 60 auf.

Auf einer der Unterseite abgekehrten Basis 61a , die im Wesentlichen kreisförmig ausgebildet ist und von einem Ringbereich 62 umgeben ist, sind der erste LED-Chip 51 und der zweite LED-Chip 52 jeweils exzentrisch zu einem Mittelpunkt der Basis 61a angeordnet. Die beiden LED-Chips 51, 52 sind über entsprechende Leiterbahnen 63 auf der Basis 61a kontaktiert. Ein elektrischer Anschluss der Bestrahlungsquelle 50 ist mit 64 bezeichnet. Die Basis 61a bildet eine Vertiefung, oberhalb der auf einer Ringschulter 62a des Ringbereichs 62 eine Filterlage 55 angeordnet ist. Das LED-Gehäuse 60 bildet eine nur in eine Richtung eine Öffnung aufweisende Vertiefung aus, von dem mehrere auf dem Träger 41 angeordnet sein können.

Die Filterlage 55 filtert die von den LED-Chips 51, 52 emittierte Strahlung innerhalb vorgegebener Wellenlängen-Grenzen, die z.B. aus gesundheitlichen Gründen vorgegeben sein können. Auf einem weiter außen und weiter weg von der Basis 61a liegenden Ringflanschbereich 62b ist die Linse 53 abgestützt und umfangsmäßig klemmend gehalten. Zusätzlich kann die Linse auch auf den Ringflanschbereich aufgeklebt sein. Man erkennt, das auf der Basis 61a noch weitere Basisbereiche vorgesehen sind, in denen weitere LED-Chips platziert werden können. Man erkennt, dass nur durch die Filterlage 55 gefilterte Strahlung aus der Linse 53 austreten kann.

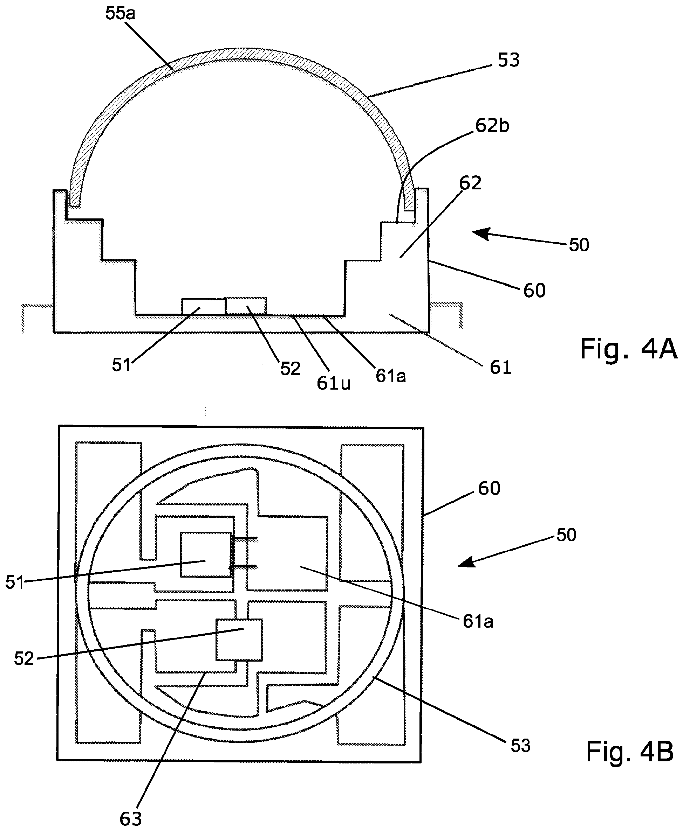

Fig. 4A und 4B zeigen einen Querschnitt und eine Draufsicht auf eine andere Ausführungsform der Bestrahlungsquelle 50. Im Unterschied zu der Ausführung gemäß Fig. 3A und 3B ist hier keine Filterlage 55 vorgesehen. Allerdings ist in die Linse 53 eine Filteranordnung 55a integriert, die die von den LED-Chips 51, 52 emittierte Strahlung innerhalb vorgegebener Wellenlängen-Grenzen filtert. Es ist möglich, die Filterlage 55 und die Filteranordnung 55a in einer Bestrahlungsquelle 50 kombiniert vorzusehen. In einer anderen Ausführung wird keinerlei Filterung der Strahlung vorgenommen.

### Beispiel 1:

Die Bestrahlungsquelle 50 der Körperbestrahlungsvorrichtung 1 umfasst einen ersten LED-Chip 51 mit einem ersten Strahlungspeak von 290 nm und einen zweiten LED-Chip 52 mit einem zweiten Strahlungspeak von 297 nm. Das Intensitätsverhältnis (Höhe) des ersten Strahlungspeaks zu dem zweiten Strahlungspeak beträgt 1:5. Die Behandlung dient der Erzeugung von Previtamin D3, wobei zugleich eine DNA-Schädigung des bestrahlten Körpers der Person 10 minimal ausfällt.

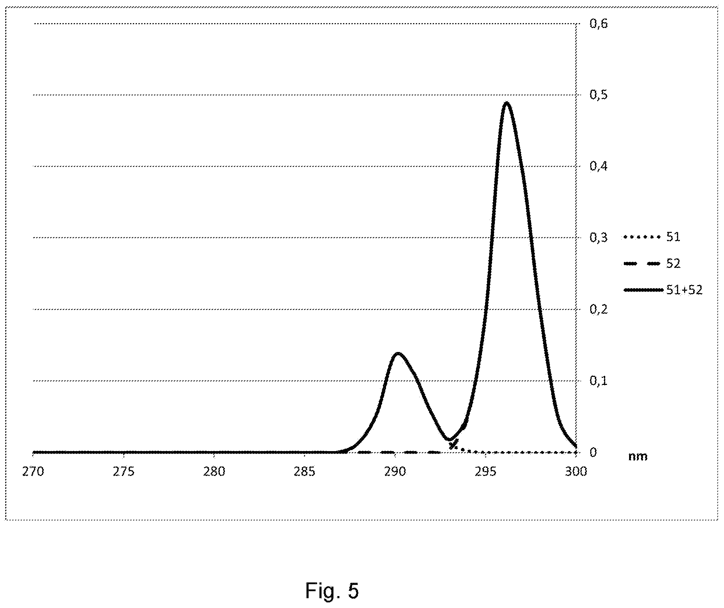

In Fig. 5 ist der Intensitäts-Verlauf des ersten Strahlungsspektrums des ersten LED-Chips 51 und des zweiten Strahlungsspektrums des zweiten LED-Chips 52 mit den vorgenannten Strahlungspeaks in einem Diagramm über der Wellenlänge dargestellt. Die resultierende Kurve ist ebenfalls eingezeichnet.

### Beispiel 2:

Die Bestrahlungsquelle 50 der Körperbestrahlungsvorrichtung 1 umfasst einen ersten LED-Chip 51 mit einem ersten Strahlungspeak von 310 nm und einen zweiten LED-Chip 52 mit einem zweiten Strahlungspeak von 365 nm. Das Intensitätsverhältnis (Höhe) des ersten Strahlungspeaks zu dem zweiten Strahlungspeak beträgt 3:1, kann aber beliebig variiert werden. Die Behandlung dient der Anregung der Pigmentierung der Haut der Person 10, allgemein auch als Bräunung bezeichnet.

### Beispiel 3:

Die Bestrahlungsquelle 50 der Körperbestrahlungsvorrichtung 1 umfasst einen ersten LED-Chip 51 mit einem ersten Strahlungspeak von 620 nm und einen zweiten LED-Chip 52 mit einem zweiten Strahlungspeak von 660 nm. Das Intensitätsverhältnis (Höhe) des ersten Strahlungspeaks zu dem zweiten Strahlungspeak beträgt 2:1, wobei die Einstellung in breiten Grenzen möglich ist. Die Behandlung dient der Hautverjüngung, der Wundheilung und kosmetischen Zwecken.

### Beispiel 4:

Die Bestrahlungsquelle 50 der Körperbestrahlungsvorrichtung 1 umfasst einen ersten LED-Chip 51 mit einem ersten Strahlungspeak von 465 nm und einen zweiten LED-Chip 52 mit einem zweiten Strahlungspeak von 660 nm und einen dritten LED-Chip (nicht dargestellt) mit einem dritten Strahlungspeak von 840 nm. Das Intensitätsverhältnis (Höhe) des ersten Strahlungspeaks zu dem zweiten Strahlungspeak zu dem dritten Strahlungspeak beträgt 1:2:1, wobei die Einstellung in breiten Grenzen möglich ist. Die Behandlung dient der Akne-Prävention und -Verringerung der Haut der Person 10.

### Beispiel 5:

Die Bestrahlungsquelle 50 der Körperbestrahlungsvorrichtung 1 umfasst einen ersten LED-Chip 51 mit einem ersten Strahlungspeak von 620 nm und einen zweiten LED-Chip 52 mit einem zweiten Strahlungspeak von 660 nm und einen dritten LED-Chip (nicht dargestellt) mit einem dritten Strahlungspeak von 740 nm und einen vierten LED-Chip (nicht dargestellt) mit einem vierten Strahlungspeak von 840 nm. Das Intensitätsverhältnis (Höhe) des ersten Strahlungspeaks zu dem zweiten Strahlungspeak zu dem dritten Strahlungspeak zu dem vierten Strahlungspeak beträgt 2:1:1:2, wobei die Einstellung in breiten Grenzen möglich ist. Die Behandlung dient der Hautverjüngung, der Wundheilung und kosmetischen Zwecken sowie insbesondere der Regeneration von Sehnen, Faszien und Muskulatur der Person 10.

### Beispiel 6:

Die Bestrahlungsquelle 50 der Körperbestrahlungsvorrichtung 1 umfasst einen ersten LED-Chip 51 mit einem ersten Strahlungspeak von 465 nm und einen zweiten LED-Chip 52 mit einem zweiten Strahlungspeak von 620 nm und einen dritten LED-Chip (nicht dargestellt) mit einem dritten Strahlungspeak von 660 nm und einen vierten LED-Chip (nicht dargestellt) mit einem vierten Strahlungspeak von 740 nm und einen fünften LED-Chip (nicht dargestellt) mit einem fünften Strahlungspeak von 840 nm. Das Intensitätsverhältnis (Höhe) des ersten Strahlungspeaks zu dem zweiten Strahlungspeak zu dem dritten Strahlungspeak zu dem vierten Strahlungspeak zu dem fünften Strahlungspeak beträgt 1:1:1:1:1, wobei die Einstellung in breiten Grenzen möglich ist. Die Behandlung dient der Hautverjüngung, der Wundheilung und kosmetischen Zwecken sowie insbesondere der Regeneration von Sehnen, Faszien und Muskulatur sowie der Akne-Prävention und -Verringerung der Haut der Person 10.

In allen vorstehend aufgeführten Beispielen kann die Bestrahlung durch einen der LED-Chips sowohl kontinuierlich als auch intermittierend erfolgen, beispielsweise gepulst. Bei der gepulsten Bestrahlung ist die Bestrahlungsdauer vorteilhaft doppelt so lange wie die Pause zwischen zwei aufeinanderfolgenden Bestrahlungen.

Aus den vorstehenden Beispielen wird deutlich, dass die gezielte Behandlung des Körpers mit Strahlung bestimmter Wellenlängen sehr vorteilhafte Wirkungen erreichen kann. Es besteht allerdings die Gefahr der Überdosierung, wenn die Person den Behandlungserfolg erzwingen möchte. Daher ist in einer vorteilhaften Ausführung vorgesehen, dass zumindest einer und vorzugsweise beide der beiden LED-Chips aus dem Beispiel 2 zusätzlich mit in der Bestrahlungsquelle 50 verbaut wird, damit die einsetzende Bräunung einem Missbrauch vorbeugt.

Man erkennt aus den vorstehenden Beispielen, dass sich bei einer Bestrahlungsquelle 50 mit beispielsweise 5 dedizierten LED-Chips eine Vielzahl von Bestrahlungskombinationen erstellen lassen, bei der je nach angestrebtem Ergebnis nicht zwangsläufig alle LED-Chips zum Einsatz kommen. So kann die Bestrahlungsquelle 50 aus Beispiel 6 auch die Bestrahlungsmuster der Beispiele 3, 4 und 5 erzeugen.

Die LED-Chips sind vorstehend jeweils nach aufsteigender Wellenlänge nummeriert worden. Es versteht sich, dass die Zuordnung zu dem ersten, zweiten usw. von mehreren LED-Chips in einer beliebigen Zuweisung erfolgen kann. So kann beispielsweise in den Beispielen 4, 5 oder 6 der Strahlungspeak von 840 nm, der außerhalb des sichtbaren Spektrums liegt, dem ersten oder zweiten LED-Chip zugeordnet werden. Bei den Beispielen mit zwei LED-Chips kann die Zuordnung zu den Strahlungspeaks beispielsweise vertauscht werden, da die Reihenfolge beliebig ist.

Die Erfindung ist vorstehend anhand von Ausführungsbeispielen erläutert worden, bei denen die Bestrahlungspeaks aus einer Gruppe mit bestimmten Wellenlängen ausgewählt worden sind. Es versteht sich, dass weitere LED-Chips mit Bestrahlungspeaks bei anderen Wellenlängen ohne Weiteres zu der Gruppe hinzugefügt werden können.

Die Erfindung ist vorstehend anhand eines Ausführungsbeispiels erläutert worden, bei dem eine Filterlage 55 zwischen den LED-Chips 51, 52 und der Linse 53 vorgesehen ist, oder bei dem die Linse 53 eine integrierte Filteranordnung 56 aufweist. Es versteht sich, dass bei einem schmalen Spektrum der von den LED-Chips erzeugten Strahlung eine Filterlage und/oder eine Filteranordnung verzichtbar ist, ebenso bei solchen LED-Chips, deren Strahlungsspektrum im sichtbaren oder Nah-Infrarot Bereich liegt.

Die Erfindung ist vorstehend anhand von Ausführungsbeispielen erläutert worden, bei denen jeweils nur ein LED-Chip mit einem bestimmten Strahlungspeak in der Bestrahlungsquelle vorgesehen ist. Es versteht sich, dass ungeachtet der weiteren LED-Chips mit anderen Strahlungspeaks auch mehr als ein LED-Chip mit dem bestimmten Strahlungspeak vorgesehen sein kann.

Die Erfindung ist vorstehend anhand eines Ausführungsbeispiels erläutert worden, bei dem der Träger 41 des Bestrahlungsmoduls 40 im Wesentlichen rechteckig ausgebildet ist und ein Feld von 4x5 Bestrahlungsquellen 50 aufweist. Es versteht sich, dass der Träger 41 auch eine andere Gestalt, beispielsweise quadratisch oder hexagonal oder linienförmig aufweisen kann, und dass die Bestrahlungsquellen 50 auch anders auf dem Träger 41 angeordnet sein können.

Die Erfindung ist vorstehend anhand eines Ausführungsbeispiels erläutert worden, bei dem der Träger 41 auf einer Wärmeübergangsplatte 46 angeschlossen ist, die über Kühlleitungen 47 mit einem Wärmetauscher verbunden ist. Es versteht sich, dass der Wärmetauscher 48 über weitere Kühlleitungen zugleich mit einem weiteren Träger 41 verbunden sein kann, und dass es auch möglich ist, mehrere Wärmeübergangsplatten über Verbindungsleitungen zu einem geschlossenen System mit dem Wärmetauscher 48 zu verbinden.

Die Erfindung ist vorstehend anhand eines Ausführungsbeispiels erläutert worden, bei dem sämtliche Bestrahlungsmodule 40 in der Vorrichtung 1 in gleicher Weise ausgebildet sind. Es versteht sich, dass die Bestrahlungsmodule 40 für den Schulter- und Kopfbereich, die Bestrahlungsmodule in dem unteren Gehäuseteil 20 und die Bestrahlungsmodule in dem oberen Gehäuseteil 30 jeweils auch verschieden ausgebildet sein können, und insbesondere auch eine unterschiedliche Anzahl von LEDs aufweisen können.

Die Erfindung ist vorstehend anhand eines Ausführungsbeispiels erläutert worden, bei dem die Bestrahlungsmodule 40 ortsfest in den Gehäuseteilen 20, 30 angeordnet sind, und im Wesentlichen in Reaktion von den ersten Sensor 61 und zweiten Sensor 62 erfassten Daten angesteuert werden. Es versteht sich, dass statt einer elektrischen Ansteuerung der Bestrahlungsmodule 40 diese auch hinsichtlich ihrer Entfernung zu dem Körper des Benutzers 10 einstellbar sein können, beispielsweise über pneumatische, hydraulische, mechanische oder elektrische Verstelleinrichtungen.

Die Erfindung ist vorstehend anhand eines Ausführungsbeispiels erläutert worden, bei dem die Vorrichtung 1 ein stationäres Unterteil 20 und ein schwenkbar auf das Unterteil 20 herabschwenkbares oberes Gehäuseteil 30 aufweist, wobei der Benutzer 10 auf einer Liegefläche 21 des unteren Gehäuseteils 20 ruht. Es versteht sich, dass die Vorrichtung auch in der Gestalt eines Stehbräuners ausgebildet sein kann, bei dem die beiden Gehäuseteile im Wesentlichen senkrecht stehend angeordnet sind, und bei dem der Benutzer während der Bestrahlung im Wesentlichen auf dem Boden steht und von dem Gehäuseteilen umgeben ist.

Die Erfindung ist vorstehend anhand eines Ausführungsbeispiels erläutert worden, bei dem ein Sensor 61, 62 Eigenschaften der Vorrichtung 1 bzw. der Person 10 erfasst. Es versteht sich, dass hierfür auch mehrere Sensoren vorgesehen sein können, und dass die von den Sensoren gewonnenen Daten auch gespeichert werden können, um die ordnungsgemäße Einstellung der Vorrichtung zu dokumentieren.

Die Erfindung ist vorstehend anhand eines Ausführungsbeispiels erläutert worden, bei dem die Bestrahlungsquellen 50 mit ihrer Basis 61a zu Bestrahlungsmodulen 40 zusammengefasst worden sind. Es versteht sich, dass es keiner Bestrahlungsmodule mit mehreren einzelnen Bestrahlungsquellen 50 auf einem gemeinsamen Träger bedarf, sondern dass jede Bestrahlungsquelle 50 auch auf einem gesonderten Träger angeordnet sein kann, wodurch sich insbesondere der gekrümmte Verlauf des oberen Gehäuseteils 30 leichter folgen lässt. Entsprechend ist dann die Bestrahlungsquelle 50 unmittelbar an der Körperbestrahlungsvorrichtung 1 angeschlossen.

## Patentansprüche

1. Körperbestrahlungsvorrichtung zur Bestrahlung eines Körpers einer Person oder eines Teils eines Körpers einer Person, insbesondere mit kosmetisch-hygienisch nützlicher Strahlung, umfassend
eine Bestrahlungsquelle (50) mit einer Basis (61a) und zumindest einem auf der Basis (61a) angeordneten ersten LED-Chip (51), der ein erstes Strahlungsspektrum mit einem ersten Strahlungspeak aussenden kann und zumindest einem zweiten LED-Chip (52), der ein zweites Strahlungsspektrum mit einem von dem ersten Strahlungspeak verschiedenen zweiten Strahlungspeak aussenden kann, wobei der erste LED-Chip (51) unter einer Linse (53) in einem LED-Gehäuse (60) angeordnet ist,
ein Gehäuse, das einen tunnelartigen Bestrahlungsraum (2) für die zu bestrahlende Person (10) zumindest teilweise umgibt und das zumindest einen Aufnahmeraum für die Bestrahlungsquellen (50) aufweist, wobei eine Trennwand des Gehäuses zwischen dem Aufnahmeraum und dem Bestrahlungsraum aus einem für die Strahlung der Bestrahlungsquellen transparenten Material ausgebildet ist, wobei die Strahlung der Bestrahlungsquellen (50) aus dem Aufnahmeraum in den Bestrahlungsraum gerichtet ist,
wobei der zumindest eine erste LED-Chip (51) und der zumindest eine zweite LED-Chip (52) separat ansteuerbar sind,
**dadurch gekennzeichnet,**
**dass** der zweite LED-Chip (52) auf der Basis (61a) unter der gemeinsamen Linse (53) in dem LED-Gehäuse (60) gemeinsam mit dem ersten LED-Chip (51) angeordnet ist, und
**dass** Strahlung mit dem ersten Strahlungspeak und Strahlung mit dem zweiten Strahlungspeak gleichmäßig durch die gemeinsame Linse (53) hindurch ausgesendet wird, sodass der Bestrahlungsraum (2) gleichmäßig mit dem ersten Strahlungsspektrum und dem zweiten Strahlungsspektrum ausgeleuchtet wird.

2. Körperbestrahlungsvorrichtung nach Anspruch 1, **dadurch gekennzeichnet, dass** zumindest ein Strahlungspeak eine Wellenlänge aufweist, die außerhalb des Spektrums des sichtbaren Lichts liegt.

3. Körperbestrahlungsvorrichtung nach einem der vorhergehenden Ansprüche, **dadurch gekennzeichnet, dass** die Bestrahlungsquelle zumindest einen dritten LED-Chip, der ein drittes Strahlungsspektrum mit einem von dem ersten Strahlungspeak und dem zweiten Strahlungspeak verschiedenen dritten Strahlenpeak aussenden kann und der mit dem zumindest einen ersten LED-Chip (51) und dem zumindest einen zweiten LED-Chip (52) unter der gemeinsamen Linse (53) in dem LED-Gehäuse (60) angeordnet ist und separat von diesen ansteuerbar ist, umfasst.

4. Körperbestrahlungsvorrichtung nach Anspruch 3, **dadurch gekennzeichnet, dass** die Bestrahlungsquelle (50) zumindest einen vierten LED-Chip, der ein viertes Strahlungsspektrum mit einem von dem ersten Strahlungspeak und dem zweiten Strahlungspeak und dem dritten Strahlungspeak verschiedenen vierten Strahlungspeak aussenden kann und der mit dem zumindest einen ersten LED-Chip (51) und dem zumindest einen zweiten LED-Chip (52) und dem zumindest einen dritten LED-Chip unter der gemeinsamen Linse (53) in dem LED-Gehäuse (60) angeordnet ist und separat von diesen ansteuerbar ist, umfasst.

5. Körperbestrahlungsvorrichtung nach Anspruch 4, **dadurch gekennzeichnet, dass** die Bestrahlungsquelle (50) zumindest einen fünften LED-Chip, der ein fünftes Strahlungsspektrum mit einem von dem ersten Strahlungspeak und dem zweiten Strahlungspeak und dem dritten Strahlungspeak und dem vierten Strahlungspeak verschiedenen fünften Strahlungspeak aussenden kann und der mit dem zumindest einen ersten LED-Chip (51) und dem zumindest einen zweiten LED-Chip (52) und dem zumindest einen dritten LED-Chip und dem zumindest einen vierten LED-Chip unter der gemeinsamen Linse in dem LED-Gehäuse (60) angeordnet ist und separat von diesen ansteuerbar ist, umfasst.

6. Körperbestrahlungsvorrichtung nach einem der vorhergehenden Ansprüche, **dadurch gekennzeichnet, dass** die LED-Chips (51; 52) auf der Basis (61a) angeordnet sind und von einer eine erste Primäroptik bildenden Linse (53) bedeckt sind.

7. Körperbestrahlungsvorrichtung nach einem der vorhergehenden Ansprüche, **dadurch gekennzeichnet, dass** zwischen den LED-Chips (51; 52) und der Linse (53) eine Filterlage (55) angeordnet ist.

8. Körperbestrahlungsvorrichtung nach einem der vorhergehenden Ansprüche, **dadurch gekennzeichnet, dass** die Linse (53) eine integrierte Filteranordnung (55a) aufweist.

9. Körperbestrahlungsvorrichtung nach einem der vorhergehenden Ansprüche, **dadurch gekennzeichnet, dass** die Strahlungspeaks der LED-Chips (51; 52) durch eine Wellenlänge definiert sind, und dass das Spektrum der LED-Chips zu mehr als 2/3, vorzugsweise zu mehr als 3/4, besonders bevorzugt um mehr als neun Zehnteln innerhalb einer Bandbreite von +/- Wellenlänge x F, wobei 0,005 < F < 0,05 um den Strahlungspeak liegt.

10. Körperbestrahlungsvorrichtung nach einem der vorhergehenden Ansprüche, **dadurch gekennzeichnet, dass** der LED-Chip (51; 52) ausgewählt ist aus der Gruppe umfassend einen Strahlungspeak mit der Wellenlänge 290 nm +/- 2 nm; 297 nm +/- 2 nm; 310 nm +/- 5 nm; 365 nm +/- 10 nm; 620 nm +/- 15 nm; 660 nm +/- 15 nm; 465 nm +/-20 nm; 840 nm +/- 20 nm; und 740 nm +/- 20 nm.

11. Körperbestrahlungsvorrichtung nach einem der vorhergehenden Ansprüche, **dadurch gekennzeichnet, dass** der Linse (53) ein Kollimations-Reflektor (44) zum Kollimieren der Strahlung nachgeordnet ist, der die Strahlung aller LED-Chips (51; 52) gleichmäßig kollimiert.

12. Körperbestrahlungsvorrichtung nach einem der vorhergehenden Ansprüche, **dadurch gekennzeichnet, dass** mehr als die Hälfte der LED-Chips (51; 52) exzentrisch zu einem durch die Projektion des Scheitels der Linse (53) auf die Basis (61a) definierten Mittelpunkt angeordnet sind.

13. Körperbestrahlungsvorrichtung nach einem der vorhergehenden Ansprüche, **dadurch gekennzeichnet, dass** zumindest einer der LED-Chips (51; 52) Strahlung gepulst aussendet.

14. Körperbestrahlungsvorrichtung nach einem der vorhergehenden Ansprüche, ferner umfassend einen Träger (41), auf dem eine Mehrzahl von Bestrahlungsquellen (50) angeordnet sind, die jeweils mehrere LED-Chips (51; 52) bedecken.

15. Körperbestrahlungsvorrichtung nach einem der vorhergehenden Ansprüche, **dadurch gekennzeichnet, dass** die Basis (61a) einen Grund des LED-Gehäuses (60) ausbildet, und dass das LED-Gehäuses (60) überdies eine ringförmige Wandung (62) aufweist und damit eine Art Kammer oder Aufnahme ausbildet.

16. Körperbestrahlungsvorrichtung nach einem der vorhergehenden Ansprüche, **dadurch gekennzeichnet, dass** auf der Basis 61a Leiterbahnen (63) vorgesehen sind, die die LED-Chips (51, 52) kontaktieren.

17. Verwendung der Körperbestrahlungsvorrichtung nach einem der vorhergehenden Ansprüche zur nicht-therapeutischen Bestrahlung einer Person mit kosmetisch-hygienisch nützlicher Strahlung aus kurzer Entfernung.

18. Verfahren zum nicht-therapeutischen Bestrahlen einer Person mit kosmetisch-hygienisch nützlicher Strahlung in einer
Körperbestrahlungsvorrichtung (1) nach einem der Ansprüche 1 bis 16, **dadurch gekennzeichnet,**
**dass** der zumindest eine erste LED-Chip (51) und der zumindest eine zweite LED-Chip (52) separat angesteuert werden.

## Claims

1. Body irradiation device for irradiating a body of a person or a part of a body of a person - in particular, with cosmetically and hygienically useful radiation - comprising
a radiation source (50) having a base (61a) and at least one first LED chip (51) arranged on the base (61a) which can emit a first radiation spectrum with a first radiation peak and at least one second LED chip (52) which can emit a second radiation spectrum with a second radiation peak different from the first radiation peak, wherein the first LED chip (51) is arranged below a lens (53) in an LED package (60),
a housing that at least partially surrounds a tunnel-like irradiation space (2) for a person (10) to be irradiated and that has at least one accommodation space for the radiation sources (50), wherein a partition wall of the housing between the accommodation space and the irradiation space is made of a material that is transparent to the radiation of the radiation sources, wherein the radiation of the radiation sources (50) is directed from the accommodation space into the irradiation space, wherein the at least one first LED chip (51) and the at least one second LED chip (52) can be controlled separately,
**characterized in**
**that** the second LED chip (52) is arranged below the lens (53) in the LED package (60) commonly with the the first LED chip (51), and
**that** radiation with the first radiation peak and radiation with the second radiation peak is emitted uniformly through the common lens (53), such that the irradiation space (2) is illuminated uniformly with the first radiation spectrum and the second radiation spectrum.

2. Body irradiation device according to claim 1, **characterized in that** at least one radiation peak has a wavelength that is outside the spectrum of visible light.

3. Body irradiation device according to one of the preceding claims, **characterized in that** the radiation source comprises at least one third LED chip which can emit a third radiation spectrum with a third radiation peak different from the first radiation peak and the second radiation peak, and which is arranged with the at least one first LED chip (51) and the at least one second LED chip (52) below the common lens (53) in the LED package (60) and can be controlled separately from them.

4. Body irradiation device according to claim 3, **characterized in that** the radiation source (50) comprises at least one fourth LED chip which can emit a fourth radiation spectrum with a fourth radiation peak different from the first radiation peak and the second radiation peak and the third radiation peak, and which is arranged with the at least one first LED chip (51) and the at least one second LED chip (52) and the at least one third LED chip below the common lens (53) in the LED package (60) and can be controlled separately from them.

5. Body irradiation device according to claim 4, **characterized in that** the radiation source (50) comprises at least one fifth LED chip which can emit a fifth radiation spectrum with a fifth radiation peak different from the first radiation peak and the second radiation peak and the third radiation peak and the fourth radiation peak, and which is arranged with the at least one first LED chip (51) and the at least one second LED chip (52) and the at least one third LED chip and the at least one fourth LED chip below the common lens in the LED package (60) and can be controlled separately from them.

6. Body irradiation device according to one of the preceding claims, **characterized in that** the LED chips (51; 52) are arranged on the base (61a) and are covered by a lens (53) forming a first primary lens.

7. Body irradiation device according to one of the preceding claims, **characterized in that** a filter layer (55) is arranged between the LED chips (51; 52) and the lens (53).

8. Body irradiation device according to one of the preceding claims, **characterized in that** the lens (53) has an integrated filter arrangement (55a).

9. Body irradiation device according to one of the preceding claims, **characterized in that** the radiation peaks of the LED chips (51; 52) are defined by a wavelength, and that the spectrum of the LED chips is more than 2/3, preferably more than 3/4, and more preferably more than nine tenths, within a bandwidth of +/- wavelength x F, wherein 0.005 < F < 0.05 is around the radiation peak.

10. Body irradiation device according to one of the preceding claims, **characterized in that** the LED chip (51; 52) is selected from the group comprising a radiation peak with the wavelength 290 nm +/- 2 nm; 297 nm +/- 2 nm; 310 nm +/- 5 nm; 365 nm +/- 10 nm; 620 nm +/- 15 nm; 660 nm +/- 15 nm; 465 nm +/- 20 nm; 840 nm +/- 20 nm; and 740 nm +/- 20 nm.

11. Body irradiation device according to one of the preceding claims, **characterized in that** a collimation reflector (44) for collimating the radiation is arranged downstream of the lens (53) and collimates the radiation of all LED chips (51; 52) uniformly.

12. Body irradiation device according to one of the preceding claims, **characterized in that** more than half of the LED chips (51; 52) are arranged eccentrically to a center point defined by the projection of the apex of the lens (53) onto the base (61a).

13. Body irradiation device according to one of the preceding claims, **characterized in that** at least one of the LED chips (51; 52) emits radiation in a pulsed manner.

14. Body irradiation device according to one of the preceding claims, further comprising a carrier (41) on which a plurality of radiation sources (50) are arranged, each covering several LED chips (51; 52).

15. Body irradiation device according to one of the preceding claims, **characterized in that** the base (61a) is a foundation of the LED package (60), and that the the LED package (60) moreover has an annular wall (62) and thus forms a type of chamber or receptacle.

16. Body irradiation device according to one of the preceding claims, **characterized in that** conductor tracks (63) are provided on the base (61a) that contact the LED chips (51, 52).

17. Use of the body irradiation device according to one of the preceding claims for the non-therapeutic irradiation of a person with cosmetically and hygienically useful radiation from a short distance.

18. Method for the non-therapeutic irradiation of a person with cosmetically and hygienically useful radiation in a body irradiation device (1) according to one of claims 1 to 16,
**characterized in**
**that** the at least one first LED chip (51) and the at least one second LED chip (52) are controlled separately.

## Revendications

1. Dispositif d'irradiation corporelle destiné à irradier le corps d'une personne ou une partie du corps d'une personne, en particulier avec un rayonnement utile sur le plan cosmétique et hygiénique, comprenant
une source de rayonnement (50) comportant une base (61a) et au moins un premier chip LED (51) disposé sur la base (61a), qui peut émettre un premier spectre de rayonnement présentant un premier pic de rayonnement, et au moins un deuxième chip LED (52) capable d'émettre un deuxième spectre de rayonnement avec un deuxième pic de rayonnement différent du premier pic de rayonnement, le premier chip LED (51) étant disposé sous une lentille (53) dans un boîtier de LED (60),
un châssis qui entoure au moins partiellement un espace d'irradiation (2) en forme de tunnel destiné à la personne (10) à irradier et qui comporte au moins un espace de réception pour les sources d'irradiation (50), une paroi de séparation du boîtier entre l'espace de réception et l'espace d'irradiation étant réalisée dans un matériau transparent au rayonnement des sources d'irradiation, le rayonnement des sources d'irradiation (50) étant dirigé de l'espace de réception vers l'espace d'irradiation,
l'au moins un premier chip LED (51) et l'au moins un deuxième chip LED (52) pouvant être commandés séparément,
**caractérisé en ce**
**que** le deuxième chip LED (52) est disposé sur la base (61a) sous la lentille commune (53) dans le boîtier de LED (60) conjointement avec le premier chip LED (51), et
**que** le rayonnement présentant le premier pic de rayonnement et le rayonnement présentant le deuxième pic de rayonnement sont émis de manière uniforme à travers la lentille commune (53), de sorte que l'espace d'irradiation (2) est éclairé de manière uniforme par le premier spectre de rayonnement et le deuxième spectre de rayonnement.

2. Dispositif d'irradiation corporelle selon la revendication 1, **caractérisé en ce qu'**au moins un pic de rayonnement présente une longueur d'onde qui se situe en dehors du spectre de la lumière visible.

3. Dispositif d'irradiation corporelle selon l'une des revendications précédentes, **caractérisé en ce que** la source d'irradiation comprend au moins un troisième chip LED, qui peut émettre un troisième spectre de rayonnement avec un troisième pic de rayonnement différent du premier pic de rayonnement et du deuxième pic de rayonnement, et qui est disposé avec l'au moins un premier chip LED (51) et l'au moins un deuxième chip LED (52) sous la lentille commune (53) dans le boîtier de LED (60) et peut être commandé séparément de ceux-ci.

4. Dispositif d'irradiation corporelle selon la revendication 3, **caractérisé en ce que** la source d'irradiation (50) comprend au moins un quatrième chip LED, qui peut émettre un quatrième spectre de rayonnement avec un quatrième pic de rayonnement différent du premier pic de rayonnement, du deuxième pic de rayonnement et du troisième pic de rayonnement, et qui est disposé avec l'au moins un premier chip LED (51), avec l'au moins un deuxième chip LED (52) et avec l'au moins un troisième chip LED sous la lentille commune (53) dans le boîtier de LED (60) et peut être commandé séparément de ceux-ci.

5. Dispositif d'irradiation corporelle selon la revendication 4, **caractérisé en ce que** la source de rayonnement (50) comprend au moins un cinquième chip LED, qui peut émettre un cinquième spectre de rayonnement avec un cinquième pic de rayonnement différent du premier pic de rayonnement, du deuxième pic de rayonnement, du troisième pic de rayonnement et du quatrième pic de rayonnement, et qui est disposé avec l'au moins un premier chip LED (51), avec l'au moins un deuxième chip LED (52), avec l'au moins un troisième chip LED et avec l'au moins un quatrième chip LED sous la lentille commune dans le boîtier de LED (60) et peut être commandé séparément de ceux-ci.

6. Dispositif d'irradiation corporelle selon l'une des revendications précédentes, **caractérisé en ce que** les chips LED (51 ; 52) sont disposés sur la base (61a) et sont recouverts par une lentille (53) formant une première optique primaire.

7. Dispositif d'irradiation corporelle selon l'une des revendications précédentes, **caractérisé en ce qu'**une couche filtrante (55) est disposée entre les chips LED (51 ; 52) et la lentille (53).

8. Dispositif d'irradiation corporelle selon l'une des revendications précédentes, **caractérisé en ce que** la lentille (53) comporte un ensemble de filtres intégré (55a).

9. Dispositif d'irradiation corporelle selon l'une des revendications précédentes, **caractérisé en ce que** les pics de rayonnement des chips LED (51 ; 52) sont définis par une longueur d'onde, et **en ce que** le spectre des chips LED se situe pour plus de 2/3, de préférence pour plus de 3/4, de manière particulièrement préférée pour plus de neuf dixièmes, dans une bande passante de +/- longueur d'onde x F, où 0,005 < F < 0,05, autour du pic de rayonnement.

10. Dispositif d'irradiation corporelle selon l'une des revendications précédentes, **caractérisé en ce que** le chip LED (51 ; 52) est choisi parmi le groupe comprenant un pic de rayonnement ayant une longueur d'onde de 290 nm ± 2 nm ; 297 nm ± 2 nm ; 310 nm ± 5 nm ; 365 nm ± 10 nm ; 620 nm ± 15 nm ; 660 nm ± 15 nm ; 465 nm ± 20 nm ; 840 nm ± 20 nm ; et 740 nm ± 20 nm.

11. Dispositif d'irradiation corporelle selon l'une des revendications précédentes, **caractérisé en ce qu'**un réflecteur de collimation (44) est disposé en aval de la lentille (53) pour collimater le rayonnement, lequel collimate uniformément le rayonnement de tous les chips LED (51 ; 52).

12. Dispositif d'irradiation corporelle selon l'une des revendications précédentes, **caractérisé en ce que** plus de la moitié des chips LED (51 ; 52) sont disposés de manière excentré par rapport à un centre défini par la projection du sommet de la lentille (53) sur la base (61a).

13. Dispositif d'irradiation corporelle selon l'une des revendications précédentes, **caractérisé en ce qu'**au moins l'un des chips LED (51 ; 52) émet un rayonnement pulsé.

14. Dispositif d'irradiation corporelle selon l'une des revendications précédentes, comprenant en outre un support (41) sur lequel sont disposées une pluralité de sources d'irradiation (50) qui recouvrent chacune plusieurs chips LED (51 ; 52).

15. Dispositif d'irradiation corporelle selon l'une des revendications précédentes, **caractérisé en ce que** la base (61a) forme un fond du boîtier de LED (60), et que le boîtier de LED (60) présente en outre une paroi annulaire (62) et forme ainsi une sorte de chambre ou de logement.

16. Dispositif d'irradiation corporelle selon l'une des revendications précédentes, **caractérisé en ce que** des pistes conductrices (63) sont prévues sur la base (61a), lesquelles sont en contact avec les chips LED (51, 52).

17. Utilisation du dispositif d'irradiation corporelle selon l'une des revendications précédentes pour l'irradiation non thérapeutique d'une personne avec un rayonnement utile sur le plan cosmétique et hygiénique à courte distance.

18. Procédé d'irradiation non thérapeutique d'une personne avec un rayonnement utile sur le plan cosmétique et hygiénique dans un dispositif d'irradiation corporelle (1) selon l'une des revendications 1 à 16, **caractérisé en ce**
**que** l'au moins un premier chip LED (51) et l'au moins un deuxième chip LED (52) sont commandés séparément.
